# EUROPEAN PATENT APPLICATION

(11) **EP 1 930 429 A1**
(43) Date of publication of application: **11.06.2008**
(21) Application number: 06782813.7
(22) Date of filing: 18.08.2006
(51) Int. Cl.: C12N 15/09, C07K 16/18, C12N 5/06, C12Q 1/02, C12Q 1/68, G01N 33/53

(54) **Msx1/2, MARKERS OF GROWING PROGENITOR CELL OF DOPAMINE-PRODUCING NEURON**

(30) Priority: 18.08.2005 JP 2005237805
(71) Applicant: Eisai R&D Management Co., Ltd., Tokyo 112-8088 (JP)
(72) Inventor: ONO, Yuichi, KAN Research Institute, Inc., Kyoto-shi, Kyoto 6008815 (JP); NAKAGAWA, Yasuko, KAN Research Institute, Inc., Kyoto-shi, Kyoto 6008815 (JP); NAKATANI, Tomoya, KAN Research Institute, Inc., Kyoto-shi, Kyoto 6008815 (JP); SAKAMOTO, Yoshimasa, KAN Research Institute, Inc., Shimogyo-ku Kyoto-shi, Kyoto 6008815 (JP)
(74) Representative: Vossius & Partner
(86) International application number: PCT/JP2006/316252
(87) International publication number: WO 2007/021004

(57) **Abstract**

The present invention is a probe, a primer, and an antibody, for detecting a dopaminergic neuron proliferative progenitor cell. According to the present invention, there is provided a polynucleotide probe and a polynucleotide primer for use in the detection or selection of a dopaminergic neuron proliferative progenitor cell, which can hybridize with a polynucleotide consisting of a nucleotide sequence of an Msx1 gene or an Msx2 gene, or a complementary sequence thereto, and an antibody against an Msx1 protein or an Msx2 protein, or a part thereof for use in the detection or selection of a dopaminergic neuron proliferative progenitor cell.

## Description

### Technical Field

The present invention relates to an Msx1 gene and an Msx2 gene, which are dopaminergic neuron proliferative progenitor cell markers. More particularly, the present invention relates to a means for detecting a dopaminergic neuron proliferative progenitor cell, a method for detecting the cell, and a kit for detecting the cell.

### Background Art

The dopamine system is a very important system involved in movement control, hormone secretion control, affectivity control, and so forth, which are important in the mammalian brain. Therefore, abnormalities in dopaminergic neurotransmission cause various disorders of the neural system. For example, the Parkinson's disease is a neurodegenerative disease of the extrapyramidal system which is caused by specific degeneration of dopaminergic neurons in the midbrain substantia nigra (HARRISON'S PRINCIPLES OF INTERNAL MEDICINE Vol. 2 23rd ed., Isselbacher et al. edited by McGraw-Hill Inc., NY (1994) pp. 2275-7).

As a method for treating the Parkinson's disease, a method of orally administering L-DOPA (3,4-dihydroxy-phenylalanine) has been mainly adopted for compensating the decrease in the amount of the produced dopamine, but it is known that the duration of the effect is not good.

Accordingly, as a method for compensating the loss of dopaminergic neurons, recently, there has been attempted a therapeutic method of transplanting a midbrain ventral region of a 6-9 week aborted fetus cotaining dopaminergic neuron precursors (US Patent No. 5690927; Spencer et al. (1992) N. Engl. J. Med. 327:1541-8; Freed et al. (1992) N. Engl. J. Med. 327:1549-55; Widner et al. (1992) N. Engl. J. Med. 327:1556-63; Kordower et al. (1995) N. Engl. J. Med. 332:1118-24; Defer et al.(1996) Brain 119:41-50; and Lopez-Lozano et al. (1997) Transp. Proc. 29:977-80). However, at the present time, in addition to cell supply and ethical issues (Rosenstain (1995) Exp. Neurol.33:106; Turner et al. (1993) Neurosurg. 33:1031-7), various other problems have been indicated, for example, risk of infectious contamination, immunologic transplant rejection (Lopez-Lozano et al. (1997) Transp. Proc. 29:977-80 and Widner and Brudin(1988) Brain Res. Rev. 13:287-324), low survival rate due to the fetus tissue's mainly dependence on lipid metabolism rather than glycolysis (Rosenstein (1995) Exp. Neurol. 33:106), and so forth.

As a method for solving the problem of the ethical issues or supply shortage, for example, a method by using a cortex, a striation, and midbrain cells, derived from a pig, and so forth have been proposed (for example, Japanese Patent Laid-Open Publication No. 10-508487, No. 10-508488, and No. 10-509034). However, in this method, a complex procedure for modifying an antigen on the cell surface (MHC class I antigen) is required to suppress rejection. As a method for solving the transplant rejection, for example, a method involving local immunosuppression by simultaneously transplanting Sertoli cells has been proposed (Japanese Patent Laid-Open Publication No. 11-509170 and No. 11-501818; and Selawly and Cameron (1993) Cell Transplant 2:123-9). It is possible that transplant cells are obtained from a relative whose MHC matches, bone marrow of another person, a bone marrow bank, a cord blood bank, and so forth. However, if patient's own cells can be used, the problems of rejection can be solved without extra procedures and trouble.

Accordingly, it has been expected that, instead of cells derived from an aborted fetus, a differentiation system of dopaminergic neurons *in vitro* from non-neural cells such as embryo-stem (ES) cell and bone marrow stromal cells are utilized as a transplant material. Actually, there is a report that a functional dopaminergic neuron is formed by ES cell transplantation into lesion striation of a rat Parkinson's disease model (Kim et al. (2002) Nature 418:50-56). It is thought that in the future, importance of regenerative medicine from ES cells or the patient's own neural stem cells will increase.

On the other hand, in the treatment of damage of neural tissue, restructuring of brain function is required, and for forming appropriate linkage with surrounding cells (network formation), not mature cells but progenitor cells that can differentiate into neurons *in vivo* are required to be transplanted. However, in the transplantation of neuron progenitor cells, in addition to the above-described problem regarding supply, there is a problem that the progenitor cells can differentiate into a nonuniform cell population. For example, in the treatment of the Parkinson's disease, it is necessary that dopaminergic neurons are selectively transplanted among catecholamine-containing neurons. Before now, as transplant cells for use in the treatment of the Parkinson's disease, there has been proposed a striate body (Lindvall et al. (1989) Arch. Neurol. 46:615-31 and Widner et al. (1992) N. Engl. J. Med. 327:1556-63), an immortalized cell line derived from human embryonic nerve (Japanese Patent Laid-Open Publication No. 8-509215, No. 11-506930, and No. 2002-522070), a post-mitotic human neuron of NT2Z cells (Japanese Patent Laid-Open Publication No. 9-5050554), a neuron primordial cell (Japanese Patent Laid-Open Publication No. 11-509729), a cell transfected with an exogenous gene so as to produce catecholamine such as dopamine, a bone marrow stromal cell (Japanese Patent Laid-Open Publication No. 2002-504503 and No. 2002-513545), an ES cell in which a gene is modified (Kim et al. (2002) Nature 418:50-56), and so forth. However, none of these contain only dopaminergic neurons or cells to differentiate into dopaminergic neurons.

As a method for selectively condensing or isolating dopaminergic neurons from undifferentiated cell population, there has been proposed a method of, introducing a reporter gene expressing a fluorescent protein under control of promoter/enhancer of a gene such as tyrosine hydroxylase (TH) expressed in dopaminergic neurons into each cell of the cell population, isolating the cells emitting fluorescence, and thereby visualizing the alive dopaminergic neurons to condense, segregate or identify (Japanese Patent Laid-Open Publication No. 2002-51775). However, this method requires a complex step of introduction of an exogenous gene, and furthermore, when used in gene treatment, the existence of the reporter gene causes problem of toxicity and immunogenicity.

As described above, now, one of the largest problems in transplantation treatment for the Parkinson's disease is that the either dopaminergic neuron progenitor cells derived from the midbrain ventral region of aborted fetus or induced to differentiate are a mixture of various cells. It is desirable that only a desired cell species is isolated and used, considering safety in neural network formation. Furthermore, considering survival or ability for correctly forming a network in a brain in which the cells are transplanted, it can be said that it is desirable from the treatment effect that earlier proliferative progenitor cells are isolated and transplanted.

Before now, as a gene that selectively expresses in the dopaminergic neuron proliferative progenitor cells, Lrp4 (WO 2004/065599) has been reported. Additionally, some markers of dopaminergic neuron precursors have been reported (WO 2004/038018 and WO 2004/052190). Among them, with respect to Lmxla, expression has been confirmed in human and mouse dopaminergic neuron proliferative progenitor cells, postmitotic dopaminergic neuron precursors, and dopaminergic neurons (WO 2005/052190).

An Msx gene is a homeobox gene involved in organ formation, and it is known that Msx1, Msx2, and Msx3 exist in mice, and Msx1 and Msx2 exist in humans (Davidson, D. (1995). Trends Genet 11:405-411). Before now, it has been reported that Msx1 is expressed in various brain cells (Ramos, C., et al. (2004). Dev Dyn 230:446-60). It has also been reported that in Msx1/Msx2 mutant mice, the expression of Wnt1 involved in patterning in the dorsoventral direction of a neural tube disappears completely in the dorsal midline of diencephalon and rostral midbrain (Bach, A., et al. (2003). Development 130:4025-36). Moreover, it has been reported that Msx1 is involved in development in the early stage of the neural tube formation (Liu, Y., et al. (2004). Development 131:1017-28).

However, it is not reported that Msx1 and Msx2 are selectively expressed in dopaminergic neuron proliferative progenitor cells.

### Summary of the Invention

The present inventors have recently found that an Msx1 gene and an Msx2 gene (hereinafter, occasionally referred to as merely "Msx1" and "Msx2") are selectively expressed in dopaminergic neuron proliferative progenitor cells. The present invention is based on this finding.

An object of the present invention is to provide a means for detecting a dopaminergic neuron proliferative progenitor cell, a method for detecting a dopaminergic neuron proliferative progenitor cell, and a kit for detecting a dopaminergic neuron proliferative progenitor cell.

Further, an object of the present invention is to provide a method for screening for a substance effective for inducing differentiation into dopaminergic neuron proliferative progenitor cells.

Furthermore, an object of the present invention is to provide a method for producing a dopaminergic neuron proliferative progenitor cell for use in the treatment of the Parkinson's disease.

The present invention provides a polynucleotide probe or polynucleotide primer for use in the detection or selection of a dopaminergic neuron proliferative progenitor cell, which can hybridize with a polynucleotide consisting of a nucleotide sequence of an Msx1 gene or an Msx2 gene, or a complementary sequence thereto (hereinafter, occasionally referred to as "probe according to the present invention" or "primer according to the present invention").

The present invention provides an antibody against an Msx1 protein or an Msx2 protein, or a part thereof for use in the detection or selection of a dopaminergic neuron proliferative progenitor cell (hereinafter, occasionally referred to as "antibody according to the present invention").

The present invention also provides a method for detecting or selecting a dopaminergic neuron proliferative progenitor cell, comprising the step of detecting expression of an Msx1 gene or an Msx2 gene (hereinafter, occasionally referred to as "detection method according to the present invention").

The present invention further provides a kit for detecting or selecting a dopaminergic neuron proliferative progenitor cell comprising at least a probe according to the present invention, a primer according to the present invention, or an antibody according to the present invention.

The present invention provides a method for screening for a substance effective for inducing differentiation into a dopaminergic neuron proliferative progenitor cell, comprising the step of detecting expression of an Msx1 gene or an Msx2 gene.

The present invention provides a method for producing a dopaminergic neuron proliferative progenitor cell for use in the treatment of the Parkinson's disease.

The present invention provides use of a polynucleotide that can hybridize with a polynucleotide consisting of a nucleotide sequence of an Msx1 gene or an Msx2 gene, or a complementary sequence thereto, for detecting or selecting a dopaminergic neuron proliferative progenitor cell.

The present invention provides use of an antibody against an Msx1 protein or an Msx2 protein, or a part thereof, for detecting or selecting a dopaminergic neuron proliferative progenitor cell.

The probe according to the present invention, the primer according to the present invention, and the antibody according to the present invention can be utilized as selective markers for dopaminergic neuron proliferative progenitor cells. Accordingly, the present invention is extremely useful in a purity test of a transplant material and development of a method for inducing differentiation into dopaminergic neuron proliferative progenitor cells in vitro, or the like, and is expected to contribute to the promotion of practical application of regenerative medicine.

### Brief Description of the Drawings

Figure 1 shows an expression period of dopaminergic neurons-related marker genes.
Figure 2 shows a midbrain of a 12.5-day mouse embryo. The midbrain is divided into four regions along the dorsoventral axis (V: most ventral region, VL: ventral lateral region, DL: dorsal lateral region, D: most dorsal region)
Figure 3 shows the results of analyzing, by the RT-PCR method, mRNA expression of Msx1, Msx2, DAT, Lmx1a and Lrp4 in each of the regions of the midbrain.
Figure 4 shows the results of analyzing, by the *in situ* hybridization, mRNA expression of Msx1, Nurr1, and TH in the midbrain of an 11.5-day mouse embryo. Dotted lines represent a region in which dopaminergic neurons are generated, and dash lines represent borders between VZ (ventricular zone) and ML (mantle layer).
Figure 5 shows the results of analyzing, by an immunostaining method, protein expression of Msx1/2 and Lmx1a, and coexpression thereof (double staining) in the midbrain of an 11.5-day mouse embryo.
Figure 6 shows the results of analyzing, by an immunostaining method, protein expression of Msx1/2, Lmx1a and TH in the ventral region of central nervous system of an 11.5-day mouse embryo.
Figure 7 shows the results of analyzing, by the RT-PCR method, expressions of Msx1, Msx2, and other dopaminergic neuron marker genes in dopaminergic neuron progenitor cells induced to differentiate from ES cells.
Figure 8 shows separation of Lrp4 positive cells and negative cells by a cell sorter.
Figure 9 shows the results of analyzing, by the RT-PCR method, expressions of Msx1, Msx2, and other dopaminergic neuron marker genes in each of the dopaminergic neuron progenitor cells separated from the cells derived from the midbrain of a 12.5-day mouse embryo (E12.5) and the SDIA differentiation induction cells.

### Detailed Description of the Invention

Hereinafter, the present invention will be explained in detail. The following description is an example for explaining the present invention and the present invention is not limited to the embodiments to be described. All technical terms, scientific terms, and terminologies used in the present specification have the same meanings as those that are generally understood by those ordinary skilled in the art in the technical fields to which the present invention belongs and are used merely for the purpose of explanation of a specific embodiment but are not intended to make limitation. The present invention can be carried out in various embodiments as long as not departing from the spirit thereof. All the prior art documents, published publications, patent publications, and other patent documents, cited in the present specification, are incorporated into the present specification as references, and can be used for carrying out the present invention.

### [Dopaminergic Neuron Proliferative Progenitor Cell]

The "dopaminergic neuron proliferative progenitor cell", which is an object to be detected or selected in the present invention, means a dopaminergic neuron progenitor cell before arrest of mitotic division.

The dopaminergic neuron differentiates from a neuroepithelial cell, through differentiation stages of a proliferative progenitor cell and a postmitotic precursor cell, into a mature dopaminergic neuron. The dopaminergic neuron proliferative progenitor cell is the earliest progenitor cell in the dopaminergic neurons, and therefore, high survival rate and high ability of network formation in the brain to which the cell is transplanted can be expected. Therefore, the dopaminergic neuron proliferative progenitor cell is useful for transplantation therapy for diseases caused by decrease in dopamine due to degeneration of the dopaminergic neurons such as the Parkinson's disease.

The cells selected by using the probe, the primer, or the antibody according to the present invention as an index are dopaminergic neuron proliferative progenitor cells before arrest of mitotic division, and therefore, are preferable for the transplantation treatment for neurodegenerative diseases such as the Parkinson's disease in the aspects of safety, survival rate, and network formation ability, compared to a conventional mixed cell population or dopaminergic neuron precursors in which an exogenous gene is introduced. The cells are dopaminergic neuron proliferative progenitor cells before arrest of mitotic division, namely, in proliferation, and have the possibility of differentiating to mature in the most appropriate place in the brain, and also, the dopaminergic neuron progenitor cells have the possibility of proliferating *in vivo,* and therefore, a longer effect of the treatment can be expected. Therefore, it can be said that the present invention paves the way to the practical application of the effective transplantation treatment of neurodegenerative diseases such as the Parkinson's disease.

### [Polynucleotide Probe and Polynucleotide Primer]

The probe and the primer according to the present invention can hybridize specifically with an Msx1 gene or an Msx2 gene. As described above, the expression of an Msx1 gene or an Msx2 gene is useful as an index of dopaminergic neuron proliferative progenitor cells. Therefore, the probe or the primer according to the present invention can be used as a marker for detecting dopaminergic neuron proliferative progenitor cells.

The probe and the primer according to the present invention can be used for detecting expression of an Msx1 gene or an Msx2 gene, and corresponds to a polymer consisting of a plurality of bases or base pairs such as deoxyribonucleic acid (DNA) or ribonucleic acid (RNA). It is known that double-strand cDNA can also be used in tissue *in situ* hybridization, and such double strand cDNA is included in the probe and the primer according to the present invention. As a particularly preferable probe and primer in detection of RNA in tissue, an RNA probe (riboprobe) can be exemplified.

The probe and the primer according to the present invention includes those consisting of a polynucleotide comprising a sequence of at least 10, preferably at least 15, more preferably at least 20, and further preferably at least 25 contiguous nucleotides of a nucleotide sequence of an Msx1 gene or an Msx2 gene, or a complementary sequence thereto. Also, the probe and the primer according to the present invention includes those consisting of a polynucleotide comprising a sequence of 10-50 or 10-30, 15-50 or 15-30, 20-50 or 20-30, and 25-50 or 25-30 contiguous nucleotides of a nucleotide sequence of an Msx1 gene or an Msx2 gene, or a complementary sequence thereto.

The probe and the primer according to the present invention can be at least 10 base length, preferably at least 15 base length, more preferably at least 20 base length, further preferably at least 25 base length. The probe and the primer according to the present invention can also be 10-50 base length or 10-30 base length, 15-50 base length or 15-30 base length, 20-50 base length or 20-30 base length, and 25-50 base length or 25-30 base length.

Preferable embodiments of the probe and the primer according to the present invention provide a probe and a primer having 15-30 base length for use in the detection or selection of a dopaminergic neuron proliferative progenitor cell, consisting of a polynucleotide comprising a sequence of at least 10 (preferably at least 15, more preferably at least 20, and further preferably at least 25) contiguous nucleotides of a nucleotide sequence of an Msx1 gene or an Msx2 gene, or a complementary sequence thereto, which can hybridize with an Msx1 gene or an Msx2 gene.

Preferable embodiments of the probe and the primer according to the present invention provide those that can hybridize with a high discrimination part in the nucleotide sequence of an Msx1 gene or an Msx2 gene. By using such a probe and a primer, it becomes possible to detect the proliferative progenitor cells with higher accuracy. Such a probe and a primer include those that can hybridize with a nucleotide sequence comprising a part or all of a nucleotide sequence selected from the group consisting of nucleotides 1-710 and 963-1713 of SEQ ID NO:1, nucleotides 1-677 and 943-1546 of SEQ ID NO:3, nucleotides 1-484 and 736-1316 of SEQ ID NO:5, nucleotides 1-612 and 864-1801 of SEQ ID NO:7, nucleotides 1-737 and 976-1724 of SEQ ID NO:9, nucleotides 1-525 and 777-1189 of SEQ ID NO:11, nucleotides 1-612 and 864-1810 of SEQ ID NO:13, nucleotides 1-754 and 994-1754 of SEQ ID NO:15, nucleotides 1-744 and 996-1935 of SEQ ID NO:16, nucleotides 1-610 and 864-1806 of SEQ ID NO:17, nucleotides 1-610 and 864-1785 of SEQ ID NO:19, nucleotides 1-1456 and 1710-1905 of SEQ ID NO:21, nucleotides 1-625 and 891-1062 of SEQ ID NO:23, nucleotides 1-709 and 963-1895 of SEQ ID NO:25, nucleotides 1-520 and 786-1310 of SEQ ID NO:27, nucleotides 1-510 and 767-1259 of SEQ ID NO:29, nucleotides 1-473 and 712-2180 of SEQ ID NO:31, nucleotides 1-468 and 707-1138 of SEQ ID NO:33, nucleotides 1-435 and 674-1143 of SEQ ID NO:35, nucleotides 1-473 and 712-1164 of SEQ ID NO:37, nucleotides 1-473 and 712-1164 of SEQ ID NO:39, nucleotides 1-473 and 712-2048 of SEQ ID NO:41, nucleotides 1-473 and 712-2182 of SEQ ID NO:43, nucleotides 1-413 and 651-804 of SEQ ID NO:45, nucleotides 1-431 and 670-2605 of SEQ ID NO:47, nucleotides 1-435 and 674-2136 of SEQ ID NO:49, nucleotides 1-778 and 1015-1452 of SEQ ID NO:51, nucleotides 1-780 and 1017-1453 of SEQ ID NO:53, nucleotides 1-370 and 609-800 of SEQ ID NO:55, nucleotides 1-29 and 267-420 of SEQ ID NO:57, nucleotides 1-1340 and 1578-1731 of SEQ ID NO:59, nucleotides 1-541 and 778-2225 of SEQ ID NO:61, nucleotides 1-404 and 651-804 of SEQ ID NO:63, nucleotides 1-632 of SEQ ID NO:65, nucleotides 1-489 and 728-1107 of SEQ ID NO:67, and nucleotides 1-487 and 708-2569 of SEQ ID NO:69

The probe according to the present invention can be used as a probe according to the general methods in known methods for detecting a gene of interest such as a northern blotting method, a southern blotting method, an *in situ* hybridization method, and so forth.

The probe according to the present invention can be chemically synthesized based on the nucleotide sequences disclosed in the present specification. The preparation of the probe is well-known and can be performed, for example, according to "Molecular Cloning, A Laboratory Manual 2nd ed."(Cold Spring Harbor Press (1989)) and "Current Protocols in Molecular Biology" (John Wiley & Sons (1987-1997)).

The primer according to the present invention can also be used as a primer set consisting of two or more kinds of the primers.

The primer and the primer set according to the present invention can be utilized as a primer and a primer set according to the general methods in known methods for detecting a gene of interest by utilizing a nucleic acid amplification method such as a PCR method, a RT-PCR method, a real-time PCR method, an *in situ* PCR method, or a LAMP method.

The primer set according to the present invention can be selected so that the nucleotide sequence of an Msx1 gene or an Msx2 gene can be amplified by a nucleic acid amplification method. Nucleic acid amplification methods are well-known, and selection of the primer pair in the nucleic acid amplification method is understood by those skilled in the art. For example, in the PCR method, primers can be selected so that one of the two primers (primer pair) is paired with the plus strand of the double strand DNA of an Msx1 gene or an Msx2 gene, the other primer is paired with the minus strand of the double strand DNA, and with a strand extended by one primer, the other primer can be paired. Moreover, in the LAMP method (WO 00/28082), with respect to the target gene, three regions F3c, F2c, and F1c are defined from the 3' end side, and three regions B1, B2, and B3 are defined from the 5' end side, and by using the six regions, four kinds of primers can be designed.

The primer according to the present invention can be chemically synthesized based on the nucleotide sequences disclosed in the present specification. The preparation of the probe is well-known and can be performed, for example, according to "Molecular Cloning, A Laboratory Manual 2nd ed." (Cold Spring Harbor Press (1989)), "Current Protocols in Molecular Biology" (John Wiley & Sons (1987-1997)).

In the present invention, the "Msx1 gene", which is an index of the existence of the dopaminergic neuron proliferative progenitor cell, is known in human, mouse, rat, chimpanzee, dog, bovine, chicken, and so forth, and "Msx2 gene" is known in human, mouse, rat, chimpanzee, dog, bovine, chicken, quail, and so forth. GenBank Accession Numbers disclosing the respective sequences are as follows.

### Msx1 Gene

Human: NM_002448(SEQ ID NO:1(base sequence), SEQ ID NO:2(amino acid sequence), hereinafter representation will be in the same order), BC021285(SEQ ID NO:3, SEQ ID NO:4), M97676(same as NM_002448), and BC067353(SEQ ID NO:5, SEQ ID NO:6)
Mouse: NM_010835(SEQ ID NO:7, SEQ ID NO:8), BC016426(SEQ ID NO:9, SEQ ID NO:10), AF308572(SEQ ID NO:11, SEQ ID NO:12), X14759(SEQ ID NO:13, SEQ ID N0:14), AK078600(SEQ ID NO:15(base sequence)), AK077524(SEQ ID NO:16(base sequence)), and X59251(same as NM_010835)
Rat: NM_031059(SEQ ID NO:17, SEQ ID NO:18) and D83036(SEQ ID NO:19, SEQ ID NO:20)
Chimpanzee: XM_517087(SEQ ID NO:21, SEQ ID NO:22)
Dog: XM_545946(SEQ ID NO:23, SEQ ID NO:24)
Bovine: NM_174798(SEQ ID NO:25, SEQ ID NO:26) and D30750(same as NM_174798)
Chicken: NM_205488(SEQ ID NO:27, SEQ ID NO:28), D10372(same as NM_205488), and M76985(SEQ ID NO:29, SEQ ID NO:30)
Msx2 Gene
Human: NM_002449(SEQ ID NO:31, SEQ ID NO:32), CR592938 (SEQ ID NO:33, SEQ ID NO:34), BC015509(SEQ ID NO:35, SEQ ID NO:36), D31771(SEQ ID NO:37, SEQ ID NO:38), S75308(SEQ ID NO:39, SEQ ID NO:40), S75361(SEQ ID NO:41, SEQ ID NO:42), D89377(SEQ ID NO:43, SEQ ID NO:44), BT009814(SEQ ID NO:45, SEQ ID NO:46), X69295(SEQ ID NO:47, SEQ ID NO:48), and D26145(SEQ ID NO:49, SEQ ID NO:50)
Mouse: NM_013601(SEQ ID NO:51, SEQ ID NO:52), and X59252(SEQ ID NO:53, SEQ ID NO:54)
Rat: U12514(SEQ ID NO:55, SEQ ID NO:56), and NM_012982(SEQ ID NO:57, SEQ ID NO:58)
Chimpanzee: XM_523807(SEQ ID NO: 59, SEQ ID NO:60)
Dog: NM_001003098(SEQ ID NO:61, SEQ ID NO:62) and AJ277753(SEQ ID NO:63, SEQ ID NO:64)
Bovine: XM_592489(SEQ ID NO:65, SEQ ID NO:66)
Chicken: NM_204559(SEQ ID NO:67, SEQ ID NO:68), and S64478(same as NM_204559)
Quail: M57611(SEQ ID N0:69, SEQ ID NO:70)
Also with respect to an animal (preferably mammal) except for the above-described animals, those skilled in the art can specify a sequence of an Msx1 gene or an Msx2 gene inherent in the animal, based on the known full-length sequence of an Msx1 gene or an Msx2 gene. For example, by homology search based on the human or mouse Msx1 gene or Msx2 gene, an Msx1 gene or an Msx2 gene of the animal can be searched and identified. In the homology search, BLAST to be described later or the like can be used.

The Msx1 gene includes:
a polynucleotide encoding a human Msx1 protein consisting of an amino acid sequence of at least one selected from the group consisting of SEQ ID NO:2, SEQ ID NO:4, and SEQ ID NO:6;
a polynucleotide encoding a mouse Msx1 protein consisting of an amino acid sequence of at least one selected from the group consisting of SEQ ID NO:8, SEQ ID NO:10, SEQ ID N0:12, and SEQ ID NO: 14;
a polynucleotide encoding a rat Msx1 protein consisting of an amino acid sequence of at least one selected from the group consisting of SEQ ID NO:18 and SEQ ID NO:20;
a polynucleotide encoding a chimpanzee Msx1 protein consisting of an amino acid sequence of SEQ ID NO:22;
a polynucleotide encoding a dog Msx1 protein consisting of an amino acid sequence of SEQ ID NO:24;
a polynucleotide encoding a bovine Msx1 protein consisting of an amino acid sequence of SEQ ID NO:26; and
a polynucleotide encoding a chicken Msx1 protein consisting of an amino acid sequence of at least one selected from the group consisting of SEQ ID NO:28 and SEQ ID NO:30.

Moreover, the Msx1 gene includes:
a polynucleotide comprising a human Msx1 gene DNA sequence of at least one selected from the group consisting of SEQ ID NO:1, SEQ ID NO:3, and SEQ ID NO:5;
a polynucleotide comprising a mouse Msx1 gene DNA sequence of at least one selected from the group consisting of SEQ ID NO:7, SEQ ID NO:9, SEQ ID NO:11, SEQ ID NO:13, SEQ ID NO:15, and SEQ ID NO:16;
a polynucleotide comprising a rat Msx1 gene DNA sequence of at least one selected from the group consisting of SEQ ID NO:17 and SEQ ID NO:19;
a polynucleotide comprising a chimpanzee Msx1 gene DNA sequence of SEQ ID NO:21;
a polynucleotide comprising a dog Msx1 gene DNA sequence of SEQ ID NO:23;
a polynucleotide comprising a bovine Msx1 gene DNA sequence of of SEQ ID NO:25; and
a polynucleotide comprising a chicken Msx1 gene DNA sequence of at least one selected from the group consisting of SEQ ID N0:27 and SEQ ID NO:29.

The Msx2 gene includes:
a polynucleotide encoding a human Msx2 protein consisting of an amino acid sequence of at least one selected from the group consisting of SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:48, and SEQ ID NO:50;
a polynucleotide encoding a mouse Msx2 protein consisting of an amino acid sequence of at least one selected from the group consisting of SEQ ID NO:52 and SEQ ID NO:54;
a polynucleotide encoding a rat Msx2 protein consisting of an amino acid sequence of at least one selected from the group consisting of SEQ ID NO:56 and SEQ ID NO:58;
a polynucleotide encoding a chimpanzee Msx2 protein consisting of an amino acid sequence of SEQ ID NO:60;
a polynucleotide encoding a dog Msx2 protein consisting of an amino acid sequence of at least one selected from the group consisting of SEQ ID NO:62 and SEQ ID NO:64;
a polynucleotide encoding a bovine Msx2 protein consisting of an amino acid sequence of SEQ ID NO:66;
a polynucleotide encoding a chicken Msx2 protein consisting of an amino acid sequence of SEQ ID NO:68; and
a polynucleotide encoding a quail Msx2 protein consisting of an amino acid sequence of SEQ ID NO:70.

Moreover, the Msx2 gene includes:
a polynucleotide comprising a human Msx2 gene DNA sequence of at least one selected from the group consisting of SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:47, and SEQ ID NO:49;
a polynucleotide comprising a mouse Msx2 gene DNA sequence of at least one selected from the group consisting of SEQ ID NO:51 and SEQ ID NO:53;
a polynucleotide comprising a rat Msx2 gene DNA sequence of at least one selected from the group consisting of SEQ ID NO:55 and SEQ ID NO:57;
a polynucleotide comprising a chimpanzee Msx2 gene DNA sequence of SEQ ID NO:59;
a polynucleotide comprising a dog Msx2 gene DNA sequence of at least one selected from the group consisting of SEQ ID NO:61 and SEQ ID NO:63;
a polynucleotide comprising a bovine Msx2 gene DNA sequence of SEQ ID NO:65;
a polynucleotide comprising a chicken Msx2 gene DNA sequence of SEQ ID NO:67; and
a polynucleotide comprising a quail Msx2 gene DNA sequence of SEQ ID NO:69.

The Msx1 gene or the Msx2 gene according to the present invention includes genes encoding proteins which are functionally equivalent to an Msx1 protein or an Msx2 protein. Whether the gene is "functionally equivalent" can be determined by evaluating a biological phenomenon or function relating to the expression of an Msx1 gene or an Msx2 gene, for example, by evaluating whether the gene is selectively expressed in a dopaminergic neuron proliferative progenitor cell.

Moreover, the proteins which are functionally equivalent to an Msx1 protein or an Msx2 protein include proteins having polymorphism when the gene encoding the amino acid sequence (such as amino acid sequence of SEQ ID NO:2 and amino acid sequence of SEQ ID NO:32) has polymorphism.

The gene encoding the proteins which are functionally equivalent to the Msx1 protein includes:
a gene encoding an amino acid sequence (modified amino acid sequence) of an Msx1 protein (for example, the amino acid sequence of SEQ ID NO:2 and the amino acid sequence of SEQ ID NO:8) in which one or more amino acid residues are inserted, substituted or deleted, or are added to one or both ends in the amino acid sequence;
a gene that can hybridize under stringent conditions with a gene encoding an amino acid sequence of an Msx1 protein (for example, the amino acid sequence of SEQ ID NO:2 and the amino acid sequence of SEQ ID NO:8) ; and
a gene encoding an amino acid sequence having at least an identity of 70% or more with an amino acid sequence of an Msx1 protein (for example, the amino acid sequence of SEQ ID NO:2 and the amino acid sequence of SEQ ID NO:8).

Moreover, the gene encoding the proteins which are functionally equivalent to the Msx2 protein includes:
a gene encoding an amino acid sequence (modified amino acid sequence) of an Msx2 protein (for example, the amino acid sequence of SEQ ID NO:32 and the amino acid sequence of SEQ ID NO:52) in which one or more amino acid residues are inserted, substituted or deleted, or are added to one or both ends in the amino acid sequence;
a gene that can hybridize under stringent conditions with a gene encoding an amino acid sequence of an Msx2 protein (for example, the amino acid sequence of SEQ ID NO:32 and the amino acid sequence of SEQ ID NO:52) ; and
a gene encoding an amino acid sequence having at least an identity of 70% or more with an amino acid sequence of an Msx2 protein (for example, the amino acid sequence of SEQ ID NO:32 and the amino acid sequence of SEQ ID NO:52).

In the present specification, "one or more amino acid residues are inserted, substituted or deleted, or are added to one or both ends in the amino acid sequence" means that the modification is performed by a well-known technical method such as a site-directed mutagenesis or by substitution of a plurality of some amino acids to an extent of being naturally generated, or the like.

The modified amino acid sequence of an Msx1 protein or an Msx2 protein can be an amino acid sequence in which, for example, 1-30, preferably 1-20, more preferably 1-9, further preferably 1-5, and particularly preferably 1 or 2 amino acid(s) is/are inserted, substituted, or deleted, or is/are added to one or both of end(s) in amino acid sequence. The modified amino acid sequence can be preferably an amino acid sequence having one or more (preferably, one or several, or 1, 2, 3 or 4) conservative substitutions in the amino acid sequence of the Msx1 protein or the Msx2 protein.

The term "conservative substitutions" means that one or more amino acid residues are substituted with other chemically analogous amino acid residues so as not to substantially change protein activity. For example, the case that a certain hydrophobic residue is substituted with another hydrophobic residue and the case that a certain polar residue is substituted with another polar residue having the same charge can be exemplified. Functionally analogous amino acids which can be substituted in such a manner are known in the technical field, with respect to every amino acid. To give specific examples, the non-polar (hydrophobic) amino acid includes alanine, valine, isoleucine, leucine, proline, tryptophan, phenylalanine, methionine. The polar (neutral) amino acid includes glycine, serine, threonine, tyrosine, glutamine, asparagines, cysteine. Positively charged (basic) amino acids include arginine, histidine, and lysine. Moreover, negatively charged (acidic) amino acids include aspartic acid and glutamic acid.

In the present specification, "hybridize" means hybridization to a target polynucleotide under stringent conditions. Specifically, there can be exemplified a polynucleotide having identity of at least 70% or more, preferably 80% or more, more preferably 85% or more, further preferably 90% or more, further more preferably 95% or more, particularly preferably 98% or more, and most preferably 99% or more, with the target nucleotide sequence when calculation is performed using a parameter of default (initial setting) with homology search software such as FASTA, BLAST, Smith-Waterman [Meth. Enzym., 164, 765 (1988)]. Moreover, "under stringent conditions" can be performed according to a method of performing reaction in a hybridization buffer solution that can be generally used by those skilled in the art so that the temperature is 40-70°C, and preferably 60-65°C and performing rinsing in a rinse solution whose salt concentration is 15-300 mmol/L, and preferably 15-60 mmol/L. The temperature and the salt concentration can be appropriately adjusted according to length of the probe to be used. Furthermore, the condition when the hybridized nucleotide is rinsed can be 0.2 or 2xSSC, 0.1% SDS, and a temperature of 20-68°C. As to control of the stringent conditions (high stringency) or the mild condition (low stringency), the difference can be provided by salt concentration or temperature in rinsing. When the difference of the hybridization is provided by salt concentration, a stringent wash buffer (high stringency wash buffer) of 0.2xSSC and 0.1% SDS can be used, and a mild wash buffer (low stringency wash buffer) of 2xSSC and 0.1% SDS. Moreover, when the difference of the hybridization is provided by temperature, the temperature is 68°C in the stringent case, 42°C in the case of moderate stringency, and room temperature (20-25°C) in the mild case, and every case thereof may be performed under 0.2xSSC and 0.1% SDS.

In general, the prehybridization is performed under the same conditions as the hybridization. However, hybridization and preliminary rinsing are not limited to be performed under the same conditions.

The hybridization can be performed according to a known method. Moreover, in the case of using a commercially available library, the hybridization can be performed according to the method described in the appended instruction for use.

In the present specification, the term "identity" (occasionally referred to as homology) with respect to amino acid sequences means the degree of identity of the amino acid residues of the respective sequences between the sequences to be compared. In this case, existence of a gap and property of the amino acid are considered (Wilbur, Natl. Acad. Sci. U.S.A. 80:726-730(1993)). For calculation of the homology, BLAST (Altschul: J. Mol. Biol. 215:403-410 (1990)), FASTA (Peasron: Methods in Enzymiology 183:63-69 (1990)), or the like can be used.

The amino acid sequence having at least an identity of 70% or more with the amino acid sequence of an Msx1 protein or an Msx2 protein can be an amino acid sequence having identity of preferably 80% or more, more preferably 85% or more, further preferably 90% or more, further more preferably 95% or more, particularly preferably 98% or more, and most preferably 99% or more.

The "identity" may be a value calculated by using a homology search program known by those skilled in the art and can be calculated, for example, by using a parameter of default (initial setting) in the homology algorithm BLAST(Basic local alignment search tool) http:www.ncbi.nlm.nih.gov/BLAST/ in NCBI (National Center for Biotechnology Information).

### [Antibody]

The antibody according to the present invention can recognize specifically an Msx1 protein or an Msx2 protein. As described above, the expression of an Msx1 gene or an Msx2 gene is useful as an index for dopaminergic neuron proliferative progenitor cells. Therefore, the antibody according to the present invention can be used as a marker for detecting dopaminergic neuron proliferative progenitor cells.

An Msx1 protein or an Msx2 protein for obtaining the antibody according to the present invention may have antigenicity of Msx1 or Msx2 and includes a protein in which one or more amino acid residues are deleted, inserted, substituted, or added in an amino acid sequence of an Msx1 protein or an Msx2 protein. It is known that in such a protein, the same biological activity as the original protein is maintained (Mark et al. (1984) Proc. Natl. Acad. Sci. USA 81:5662-6; Zoller and Smith (1982) Nucleic Acids Res. 10:6487-500; Wang et al. (1984) Science 224:1431-3; and Dalbadie-McFarland et al. (1982) Proc. Natl. Acad. Sci. USA 79:6409-13). A method that one or more amino acid residues are deleted, inserted, substituted, or added in the state of maintaining the antigenicity of the original protein in a protein is known. For example, a polynucleotide encoding a mutant protein can be prepared by a site-directed mutagenesis and can be appropritately expressed (Molecular Cloning, A Laboratory Manual 2nd ed., Cold Spring Harbor Press (1989), Current Protocols in Molecular Biology, John Wiley & Sons (1987-1997); Sections 8.1-8.5; Hashimoto-Goto et al. (1995) Gene 152:271-5; Kinkel (1985) Proc. Natl. Acad. Sci. USA 82:488-92; Kramer and Fritz (1987) Method. Enzymol. 154:350-67; and Kunkel (1988) Method. Enzymol. 85:2763-6).

The antibody according to the present invention includes an antibody specific to a part of an Msx1 protein or an Msx2 protein. Specifically, an Msx1 protein or an Msx2 protein for obtaining an antibody of the present invention includes a polypeptide fragment having at least 6 amino acid residues or more (for example, 6, 8, 10, 12, or 15 amino acid residues or more) of the Msx1 protein or the Msx2 protein, as well as a polypeptide having a full-length amino acid sequence. The polypeptide fragment of the Msx1 protein or the Msx2 protein in the present specification includes every fragment as long as the fragment has antigenicity of the Msx1 protein or the Msx2 protein.

The preferable fragment includes polypeptide fragments such as the amino terminal or the carboxyl terminal of the Msx1 protein or the Msx2 protein. The antigenic determinant site of the polypeptide is estimated by a method of analyzing hydrophobicity/hydrophilicity of the amino acid sequence of the protein (Kyte-Doolittle(1982) J. Mol. Biol. 157:105-22) or a method of analyzing the secondary structure (Chou-Fasman (1978) Ann. Rev. Biochem. 47:251-76), and furthermore, confirmed by a computer program (Anal. Biochem. 151:540-6 (1985)) or a technique such as a PEPSCAN method (Japanese Patent Laid-Open Publication No. 60-500684) of synthesizing a short peptide and confirming the antigenicity.

The antibody against the Msx1 protein includes:
an antibody against a protein having an amino acid sequence of at least one selected from the group consisting of SEQ ID NO:2, SEQ ID NO:4, and SEQ ID NO:6, or a part thereof;
an antibody against a protein having an amino acid sequence of at least one selected from the group consisting of SEQ ID NO:8, SEQ ID NO:10, SEQ ID NO:12, and SEQ ID NO:14, or a part thereof;
an antibody against a protein having an amino acid sequence of at least one selected from the group consisting of SEQ ID NO:18 and SEQ ID NO:20, or a part thereof;
an antibody against a protein having an amino acid sequence of SEQ ID NO:22, or a part thereof;
an antibody against a protein having an amino acid sequence of SEQ ID NO:24, or a part thereof;
an antibody against a protein having an amino acid sequence of SEQ ID NO:26, or a part thereof; and
an antibody against a protein having an amino acid sequence of at least one selected from the group consisting of SEQ ID NO:28 and SEQ ID NO:30, or a part thereof.

The antibody against the Msx2 protein includes:
an antibody against a protein having an amino acid sequence of at least one selected from the group consisting of SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:48, and SEQ ID NO:50, or a part thereof;
an antibody against a protein having an amino acid sequence of at least one selected from the group consisting of SEQ ID NO:52 and SEQ ID NO:54, or a part thereof;
an antibody against a protein having an amino acid sequence of at least one selected from the group consisting of SEQ ID NO:56 and SEQ ID NO:58, or a part thereof;
an antibody against a protein having an amino acid sequence of SEQ ID NO:60, or a part thereof;
an antibody against a protein having an amino acid sequence of at least one selected from the group consisting of SEQ ID NO:62 and SEQ ID NO:64, or a part thereof;
an antibody against a protein having an amino acid sequence of SEQ ID NO:66, or a part thereof;
an antibody against a protein having an amino acid sequence of SEQ ID NO:68, or a part thereof; and
an antibody against a protein having an amino acid sequence of SEQ ID NO:70, or a part thereof.

Preferable embodiments of the antibody according to the present invention provide an antibody recognizing a high discrimination polypeptide portion in an Msx1 protein or an Msx2 protein. By using such an antibody, it becomes possible that the proliferative progenitor cells can be detected with higher accuracy. Such antibody includes an antibody against a high discrimination polypeptide portion in an Msx1 protein, for example, at least 6 amino acid residues or all of an amino acid sequence selected from a group consisting of amino acids 1-143 and 242-297. of SEQ ID NO:2, amino acids 1-216 and 315-370 of SEQ ID NO:4, amino acids 1-143 and 242-297 of SEQ ID NO:6, amino acids 1-149 and 248-299 of SEQ ID NO:8, amino acids 1-143 and 242-297 of SEQ ID NO:10, amino acids 1-149 and 248-303 of SEQ ID NO:12, amino acids 1-143 and 242-323 of SEQ ID NO:14, amino acids 1-143 and 242-297 of SEQ ID NO:18, amino acids 1-143 and 242-297 of SEQ ID NO:20, amino acids 1-472 and 571-634 of SEQ ID NO:22, amino acids 1-208 and 298-353 of SEQ ID NO:24, amino acids 1-143 and 242-297 of SEQ ID NO:26, amino acids 1-138 and 237-288 of SEQ ID NO:28, and amino acids 1-100 and 199-242 of SEQ ID NO:30. Also, such antibody as being capable of detecting the proliferative progenitor cells with higher accuracy includes an antibody against a polypeptide portion having high discrimination property in Msx2 protein, for example, at least 6 amino acid residues or all of an amino acid sequence selected from a group consisting of amino acids 1-120 and 218-267 of SEQ ID NO:32, amino acids 1-120 and 218-268 of SEQ ID NO:34, amino acids 1-120 and 219-267 of SEQ ID NO:36, amino acids 1-120 and 219-267 of SEQ ID NO:38, amino acids 1-120 and 219-267 of SEQ ID NO:40, amino acids 1-120 and 219-267 of SEQ ID NO:42, amino acids 1-120 and 219-267 of SEQ ID NO:44, amino acids 1-120 and 219-267 of SEQ ID NO:46, amino acids 1-120 and 219-267 of SEQ ID NO:48, amino acids 1-119 and 218-266 of SEQ ID NO:50, amino acids 1-121 and 220-268 of SEQ ID NO:52, amino acids 1-121 and 220-269 of SEQ ID NO:54, amino acids 1-9 and 99-139 of SEQ ID NO:56, amino acids 1-9 and 99-139 of SEQ ID NO:58, amino acids 1-466 and 527-576 of SEQ ID NO:60, amino acids 1-120 and 219-267 of SEQ ID NO:62, amino acids 1-120 and 219-267 of SEQ ID NO:64, amino acids 1-120 of SEQ ID NO:66, amino acids 1-112 and 211-259 of SEQ ID NO:68, and amino acids 1-112 and 211-259 of SEQ ID NO:70.

The antibody according to the present invention can be obtained from an antibody-producing cell supply institution such as the Developmental Studies Hybridoma Bank.

The antibody according to the present invention can also be obtained by using a well-known method for those skilled in the art (for example, "Current Protocols in Molecular Biology" (John Wiley & Sons (1987) and Antibodies: A Laboratory Manual, Ed. Harlow and David Lane, Cold Spring Harbor Laboratory (1988).

The antibody according to the present invention includes a polyclonal antibody, a monoclonal antibody, a chimeric antibody, a single-strand antibody (scFv), a humanized antibody, a polyspecific antibody, and antibody fragments such as Fab, Fab', F(ab')₂, Fc, and Fv.

In the case of the polyclonal antibody, the blood of a mammal in which an antigen is sensitized is extracted and serum is segregated from the blood by a known method to serve as the serum containing the polyclonal antibody.

According to need, fractions containing the polyclonal antibodies can also be further isolated.

In the case of the monoclonal antibody, antibody-producing cells obtained from the spleen or the lymph node of a mammal in which the above-described antigen is sensitized are extracted and cell-fused with myeloma cells. The obtained hybridoma is cloned and the antibody is collected from the culture to serve as the monoclonal antibody.

As the immunizing antigen, a fragment of the Msx1 protein or the Msx2 protein can be used. Alternatively, an antigen synthesized based on the above-described amino acid sequence can be used. The antigen may be used as a complex with a carrier protein. For preparation of the complex of the antigen and the carrier protein, various condensation agents such as glutaraldehyde, carbodiimide, maleimide-activated ester, or the like can be used. The carrier protein may be one generally used such as bovine serum albumin, thyroglobulin, hemocyanin, or the like and is generally performed coupling at a ratio of 1-5.

The animal to be immunized includes mouse, rat, rabbit, guinea pig, and hamster. The injection method includes subcutaneous, muscular, or intraperitoneal administration. In the administration, the antigen may be mixed with complete Freund's adjuvant or incomplete Freund's adjuvant. The administration is generally performed one time per 2-5 weeks.

The antigen-producing cells obtained from the spleen or the lymph node of the immunized animal is cell-fused with myeloma cells and isolated as hybridomas. The myeloma cells derived from mouse, rat, or human are used, and are preferably derived from the same species as the antigen-producing cells, but cells between different species are occasionally possible.

The cell fusion can be performed according to a previously known method, for example, the method disclosed in Nature, 256, 495, 1975.

The fusion accelerator includes polyethylene glycol or Sendai virus, and in general, the cell fusion can be performed by reaction for approximately 1-10 minutes so that the ratio of the number of the antigen-producing cells and the number of the myeloma cells is generally approximately 1:1-10:1, under a temperature of 20-40°C, and preferably 30-37°C by using polyethylene glycol (average molecular weight 1000-4000) having a concentration of approximately 20-50%.

For the screening of the antigen-producing hybridoma, various immunochemical methods can be used. For example, an ELISA method in which a microplate on which the Msx1 protein or the Msx2 protein is coated is used, an EIA method in which a microplate on which an anti-immunoglobulin antibody is coated is used, and an immunoblotting method in which a nitrocellulose transfer membrane is used after electrophoresing samples containing the Msx1 protein or the Msx2 protein.

From such wells, further cloning is performed by, for example, a limiting dilution method, and thereby, clones can be obtained. Selection and breeding of hybridomas are generally performed in a medium for animal cells (for example, RPMI1640) containing 10-20% bovine embryo serum to which HAT (hypoxanthine, aminopterin, and thymidine) is added. The clones obtained as described above are transplanted into the abdominal cavity of a SCID mouse to which pristine is preliminarily administered, and ascitic fluid containing the monoclonal antibody at high concentration is collected after 10-14 days to serve as a material for antibody purification. Also, the clones can be cultured and the culture can be a material for antibody purification.

For the purification of the monoclonal antibody, a previously known method may be used as the purification method of immunoglobulin, and the purification can be easily achieved, for example, by an ammonium sulfate fraction method, a PEG fraction method, an ethanol fraction method, utilization of an anion exchanger, affinity chromatography in which an Msx1 protein or an Msx2 protein is used, or the like.

The purification of the polyclonal antibody from the serum can be performed similarly.

### [Detection Method]

The expression of an Msx1 gene or an Msx2 gene serves as an index of the existence of dopaminergic neuron proliferative progenitor cells. Therefore, according to the present invention, by detecting expression of an Msx1 gene or an Msx2 gene, the dopaminergic neuron proliferative progenitor cells can be detected or selected.

The method for "detecting expression of an Msx1 gene or an Msx2 gene" used herein is not particularly limited as long as being capable of detecting the expression of the Msx1 gene or the Msx2 gene in the cell samples to be tested, and, for example, includes hybridization methods, nucleic acid amplification methods, and antigen-antibody reaction methods.

The "cell samples to be tested" used herein can be cell samples that are thought to contain the dopaminergic neuron proliferative progenitor cells, and the cells in the midbrain ventral region can be used. The cells in the midbrain ventral region can be obtained by a known method (Studer, L., et al. Nature Neurosci (1998) 1:290-295). Preferably, fetus' (preferably, human aborted fetus') or the patient's own cells of the midbrain ventral region can be used as the cell samples to be tested. Moreover, the culture cells containing dopaminergic neuron proliferative progenitor cells induced to differentiate *in vitro* can be used as the cell samples to be tested. The induction to differentiate into the dopaminergic neuron proliferative progenitor cells *in vitro* can be performed by the differentiation treatment by a known method such as an SDIA method (Kawasaki et al. Neuron (2000) 28(1):31-40) or a 5-stage method (Lee, SH., et al. Nature Biotech (2000) 18:675-579) by using, as a starting material, known ES cells (Kawasaki et al. Neuron (2000) 28(1):31-40 and Lee, SH., et al. Nature Biotech (2000) 18:675-579), bone marrow stromal cells, immortalized cell line derived from nerve (Japanese Patent Laid-Open Publication No. 8-509215, No. 11-506930, and No. 2002-522070), neuron primordial cells (Japanese Patent Laid-Open Publication No. 11-509729), or the like. Preferably, ES cells subjected to the differentiation treatment by the SDIA method can be used as the cell samples to be tested.

The "SDIA method" used herein can be performed by co-culturing the ES cells and the stromal cell line PA6 in a serum-free medium (Kawasaki et al. Neuron (2000) 28(1):31-40). Moreover, the "5-stage method" can be performed as follows. ES cells are cultured on a non-adherent culture plate under existence of the serum and thereby an embryoid body (EB) is formed, and sequentially, the EB is attached onto an adherent culture plate, and thereby, the neuron progenitor cells are selected. Finally, a growth factor such as Shh, FGF2, or FGF8 is added thereto, and thereby, dopaminergic neuron progenitor cells are induced (Lee, SH., et al. Nature Biotech (2000) 18:675-579).

According to the first embodiment of the detection method according to the present invention, the probe according to the present invention hybridizes with a nucleic acid sample (mRNA or transcript thereof), and the hybridization complex, namely, nucleotide double strand, is detected. Thus, the expression of the Msx1 gene or the Msx2 gene can be detected in the cell samples.

For the detailed procedure of the hybridization method, there can be referred to "Molecular Cloning, A Laboratory Manual 2nd ed."(Cold Spring Harbor Press (1989), particularly Sections 9.47-9.58, "Current Protocols in Molecular Biology" (John Wiley & Sons (1987-1997)), particularly, Sections 6.3 and6.4, "DNA Cloning 1: Core Techniques, A Practical Approach 2nd ed." (Oxford University (1995), particularly, Section 2.10 for the conditions).

The detection of expression of the Msx1 gene or the Msx2 gene by utilizing the hybridization method can be performed, for example, by the following steps of:
(a) contacting a polynucleotide derived from a cell sample to be tested, with the polynucleotide probe according to the present invention; and
(b) detecting a hybridization complex.

In step (a), mRNA prepared from the cell sample to be tested that is thought to contain dopaminergic neuron proliferative progenitor cells or complementary DNA (cDNA) transcribed from the mRNA, as the polynucleotide derived from the cell sample to be tested, can be contacted with the probe.

In the detection method by using a probe, the probe can be labeled. The label includes a label by utilizing radioactivity (such as ³²P, ¹⁴C, and ³⁵S), fluorescence (such as FITC, europium), an enzyme (such as peroxidase or alkaline phosphatase) reaction such as chemical coloring, or the like.

The detection of the hybridization product can be performed by using a well-known method such as northern hybridization, southern hybridization, or colony hybridization.

The cells in which the hybridization complex is detected are those expressing an Msx1 gene or an Msx2 gene, and therefore can be determined as the dopaminergic neuron proliferative progenitor cells.

According to the second embodiment of the detection method according to the present invention, the expression of the Msx1 gene or the Msx2 gene can be detected in the cell sample by amplifying nucleic acid samples (mRNA or transcript thereof) by a nucleic acid amplification method using the primer or primer set according to the present invention, and detecting the amplification product is detected.

The detection of expression of the Msx1 gene or the Msx2 gene by utilizing the nucleic acid amplification method can be performed, for example, by the following steps of:
(c) performing a nucleic acid amplification method by using a polynucleotide derived from a cell sample to be tested as a template and the polynucleotide primer or the polynucleotide primer set according to the present invention; and
(d) detecting a formed amplification product.

In step (c), mRNA prepared from the cell sample to be tested that is thought to contain dopaminergic neuron proliferative progenitor cells or complementary DNA (cDNA) transcribed from the mRNA can be used as the template.

The detection of the amplification product can be performed by using a nucleic acid amplification method such as a PCR method, a RT-PCR method, a real-time PCR method, or a LAMP method.

The cells in which the amplification product is detected are also expressing an Msx1 gene or an Msx2 gene, and therefore can be determined as the dopaminergic neuron proliferative progenitor cells.

According to the third embodiment of the detection method according to the present invention, the antibody according to the present invention and the cell sample are contacted, and the antigen-antibody reaction is detected. Thus, the expression of an Msx1 gene or an Msx2 gene can be detected in the cell sample.

The detection of expression of the Msx1 gene or the Msx2 gene by utilizing the antigen-antibody reaction can be performed, for example, by the following steps of:
(e) contacting a protein derived from a cell sample to be tested, with the antibody according to the present invention; and
(f) measuring an antigen-antibody complex.

The method for detecting the antigen-antibody reaction is well-known for the skilled person, and, for example, an Msx1 protein or an Msx2 protein can be detected in the cell sample to be tested that is thought to contain dopaminergic neuron proliferative progenitor cells by an immunological method. For the immunological method, a previously known method such as a immunohistologic staining method, an enzyme-linked immunosorbent assay, a western blotting method, an agglutination method, a competition method, or a sandwich method, can be applied to the cell sample subjected to appropriate treatment according to need, such as segregation or extraction operation of the cells. The immunohistologic staining method can be performed by, for example, a direct method by using a labeled antibody or an indirect method by using an labeled antibody against the antibody. For the labeling agent, a known labeling substance such as a fluorescent substance, a radioactive substance, an enzyme, a metal, or a pigment can be used.

The cells in which the antigen-antibody complex is detected are those expressing an Msx1 gene or an Msx2 gene, and therefore can be determined as the dopaminergic neuron proliferative progenitor cells.

For use in the treatment of the Parkinson's disease, it is desirable that the purity of the dopaminergic neuron proliferative progenitor cells is high.

The accuracy of the detection or selection of the dopaminergic neuron proliferative progenitor cells can be enhanced by performing each of the above-described detection steps not only once but repeatedly.

Therefore, according to the detection method according to the present invention, the dopaminergic neuron proliferative progenitor cells can be detected or selected with high accuracy by performing the above-described step twice or more.

Moreover, the accuracy of detection or selection of the dopaminergic neuron proliferative progenitor cells can be enhanced by using together other marker genes, preferably dopaminergic neuron proliferative progenitor cell marker genes except for the Msx1 genes and the Msx2 genes.

Therefore, according to the detection method according to the present invention, the dopaminergic neuron proliferative progenitor cells can be detected or selected with higher accuracy by using together dopaminergic neuron proliferative progenitor cell marker genes except for the Msx1 genes and the Msx2 genes, postmitotic dopaminergic neuron precursor cell marker genes or the like, and detecting not only expression of the Msx1 gene or the Msx2 gene but also expression of the other above-described marker genes.

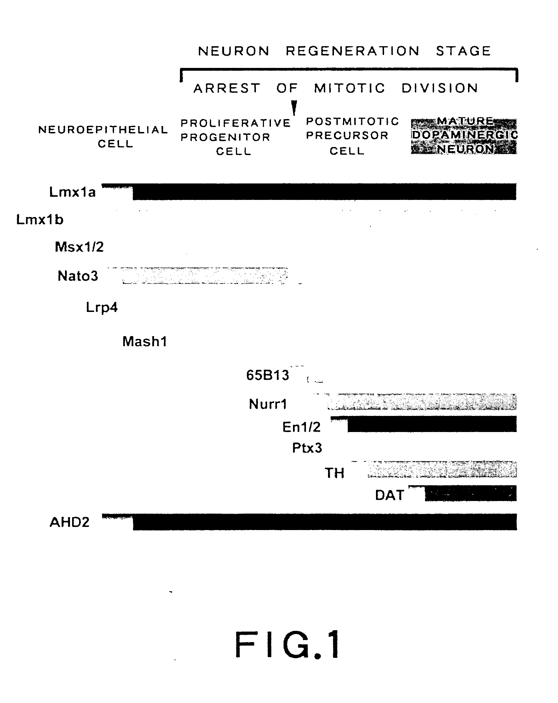

The dopaminergic neuron-related marker genes expressing selectively in each of the differentiation stages are shown in Figure 1.

In the detection method characterized in that the expression of the Msx1 gene or the Msx2 gene is detected, the dopaminergic neuron proliferative progenitor cells can be detected or selected with high accuracy by detecting not only the Msx1 gene or the Msx2 gene but also the dopaminergic neuron proliferative progenitor cell marker gene except for the Msx1 genes and the Msx2 genes by using together dopaminergic neuron proliferative progenitor cell marker gene except for the Msx1 genes and the Msx2 genes.

Specifically, in step (a), step (c), or step (e), the dopaminergic neuron proliferative progenitor cells can be detected or selected with high accuracy by using the cells in which the expression of the dopaminergic neuron proliferative progenitor cell marker gene except for the Msx1 genes and the Msx2 genes is detected as the cell sample to be tested. In this case, the cells in which the hybridization complex is detected in the step (b), the cells in which the amplification product is detected in the step (d), and the cells in which the antigen-antibody complex is detected in the step (f) each express an Msx1 gene or an Msx2 gene, and the dopaminergic neuron proliferative progenitor cell marker gene except for the Msx1 genes and the Msx2 genes. Thus, the cells can be determined as the detected or selected dopaminergic neuron proliferative progenitor cells with high accuracy.

Moreover, the dopaminergic neuron proliferative progenitor cells can be detected or selected with high accuracy by performing step (g-1) of detecting expression of the dopaminergic neuron proliferative progenitor cell marker gene except for the Msx1 genes and the Msx2 genes with respect to the cells in which the hybridization complex is detected in step (b), the cells in which the amplification product is detected in step (d), and the cells in which the antigen-antibody complex is detected in step (f), respectively. In this case, in step (g-1), the cells in which the expression of the dopaminergic neuron proliferative progenitor cell marker gene except for the Msx1 genes and the Msx2 genes is detected are those expressing an Msx1 gene or an Msx2 gene, and the dopaminergic neuron proliferative progenitor cell marker gene except for the Msx1 genes and the Msx2 genes. Thus, the cells can be determined as the detected or selected dopaminergic neuron proliferative progenitor cells with high accuracy.

In the detection method characterized in that the expression of the Msx1 gene or the Msx2 gene is detected, by using together a postmitotic dopaminergic neuron precursor cell marker gene, it can be confirmed that the Msx1 gene or the Msx2 gene is expressed but the expression of the postmitotic dopaminergic neuron precursor cell marker gene is not detected. Thus, the dopaminergic neuron proliferative precursor cells can be detected or selected with high accuracy.

Specifically, in step (a), step (c), or step (e), the dopaminergic neuron proliferative progenitor cells can be detected or selected with high accuracy by using the cells in which the expression of the postmitotic dopaminergic neuron precursor cell marker gene is not detected. In this case, the cells in which the hybridization complex is detected in step (b), the cells in which the amplification product is detected in step (d), and the cells in which the antigen-antibody complex is detected in step (f) each express an Msx1 gene or an Msx2 gene but does not express the postmitotic dopaminergic neuron precursor cell marker gene. Thus, the cells can be determined as the detected or selected dopaminergic neuron proliferative progenitor cells with high accuracy.

Moreover, the dopaminergic neuron proliferative progenitor cells can be detected or selected with high accuracy by performing step (g-2) of detecting expression of the postmitotic dopaminergic neuron precursor cell marker gene with respect to the cells in which the hybridization complex is detected in step (b), the cells in which the amplification product is detected in step (d), and the cells in which the antigen-antibody complex is detected in step (f), respectively. In this case, in step (g-2), the cells in which the expression of the postmitotic dopaminergic neuron precursor cell marker gene is not detected are those expressing an Msx1 gene or an Msx2 gene, but not the postmitotic dopaminergic neuron precursor cell marker gene. Thus, the cells can be determined as the detected or selected dopaminergic neuron proliferative progenitor cells with high accuracy.

"The dopaminergic neuron proliferative progenitor cell marker gene except for the Msx1 genes and the Msx2 genes " includes a dopaminergic neuron proliferative progenitor cell marker gene except for an Msx1 gene and an Msx2 gene which is expressed in the midbrain's most ventral ventricular region (VZ region), and includes an Lrp4 gene, a Nato3 gene, and a Mash1 gene.

An Lrp4 gene is described in WO 2004/065599. A Mash1 gene is described in Kele J, Simplicio N, Ferri AL, Mira H, Guillemot F, Arenas E, Ang SL. Neurogenin 2 is required for the development of ventral midbrain dopaminergic neurons, and Development. 2006 Feb; 133(3):495-505. It has been confirmed by the present inventors that a Nato3 gene is expressed selectively in the dopaminergic neuron proliferative progenitor cells (the data not shown).

The detection of the dopaminergic neuron proliferative progenitor cell marker gene except for the Msx1 genes and the Msx2 genes is not limited as long as using a method by which expression of the known gene can be detected, and, for example, includes the above-described hybridization method, the nucleic acid amplification method, and the antigen-antibody reaction method.

"The postmitotic dopaminergic neuron precursor cell marker gene" includes a gene expressed in the midbrain's most ventral mantle layer (ML region), and includes a Nurr1 gene, an En1 gene, an En2 gene, a Ptx3 gene, and a TH gene. Moreover, the marker gene includes a gene expressed in the midbrain's most ventral ventricular region (VZ region), and includes a 65B13 gene.

A Nurr1 gene is described in Science. 1997 11; 276(5310):248-50. An En1 gene is described in J. Neurosci. 2001 21(9): 3126-34. An En2 gene is described in J. Neurosci. 2001 21(9) 3126-34. A Ptx3 gene is described in Proc. Natl. Acad. Sci. 1997 94: 13305-10. A TH gene is described in Science 1997 11; 276(5310):248-50. A 65B13 gene is described in WO 2004/038018.

The detection of the postmitotic dopaminergic neuron precursor cell marker gene is not particularly limited as long as using a method by which expression of the known gene can be detected, and, for example, includes the above-described hybridization method, the nucleic acid amplification method, and the antigen-antibody reaction method.

### [Detection Kit]

The present invention provides a detection kit for performing the detection method according to the present invention.

The first embodiment of the detection kit according to the present invention includes a detection kit for performing the detection method of the first embodiment according to the present invention, and specifically, a kit for detecting the expression of the Msx1 gene or the Msx2 gene, including at least the probe according to the present invention. The probe may be labeled. The detection kit detects the expression of the Msx1 gene or the Msx2 gene by a hybrid formation method. Therefore, the detection method of the first embodiment can optionally further include various reagents for performing the hybrid formation method such as a substrate compound used for detection of the marker, hybridization buffer, instructions, equipment, and/or so forth.

For performing the detection with high accuracy, the detection kit of the first embodiment according to the present invention may further include the probe, the primer, the primer set, or the antibody which can detect the expression of the dopaminergic neuron proliferative progenitor cell marker gene except for the Msx1 genes and the Msx2 genes, or the expression of the postmitotic dopaminergic neuron precursor cell marker gene. The probe, the primer, the primer set, or the antibody may be labeled. By any of the hybrid formation method, the nucleic acid amplification method, and the antigen-antibody reaction method, the detection kit further detects the expression of the dopaminergic neuron proliferative progenitor cell marker gene except for the Msx1 genes and the Msx2 genes, or the expression of the postmitotic dopaminergic neuron precursor cell marker gene.

The second embodiment of the detection kit according to the present invention includes a detection kit for performing the detection method of the second embodiment according to the present invention, and specifically, a kit for detecting the expression of the Msx1 gene or the Msx2 gene, including at least the primer according to the present invention or the primer set according to the present invention. The detection kit detects the expression of the Msx1 gene or the Msx2 gene by the nucleic acid amplification method. Therefore, the detection method of the second embodiment can optionally further include various reagents for performing the nucleic acid amplification method such as a buffer, an internal standard indicating that the PCR can normally progress, instructions, equipment, and/or so forth.

For performing the detection with high accuracy, the detection kit of the second embodiment according to the present invention may further include the probe, the primer, the primer set, or the antibody which can detect the expression of the dopaminergic neuron proliferative progenitor cell marker gene except for the Msx1 genes and the Msx2 genes, or the expression of the postmitotic dopaminergic neuron precursor cell marker gene. The probe, the primer, the primer set, or the antibody may be labeled. By any of the hybrid formation method, the nucleic acid amplification method, and the antigen-antibody reaction method, the detection kit further detects the expression of the dopaminergic neuron proliferative progenitor cell marker gene except for the Msx1 genes and the Msx2 genes, or the expression of the postmitotic dopaminergic neuron precursor cell marker gene.

The third embodiment of the detection kit according to the present invention includes a detection kit for performing the detection method of the third embodiment according to the present invention, and specifically, a kit for detecting the expression of the Msx1 gene or the Msx2 gene, including at least the antibody according to the present invention. The antibody may be labeled. The detection kit detects the expression of the Msx1 gene or the Msx2 gene by detecting the antigen-antibody reaction.

Therefore, the detection method of the third embodiment can optionally further include various reagents for performing the antigen-antibody reaction such as a secondary antibody used for the ELISA method or the like, a coloring reagent, a buffer, instructions, equipment, and/or so forth.

For performing the detection with high accuracy, the detection kit of the third embodiment according to the present invention may further include the probe, the primer, the primer set, or the antibody which can detect the expression of the dopaminergic neuron proliferative progenitor cell marker gene except for the Msx1 genes and the Msx2 genes, or the expression of the postmitotic dopaminergic neuron precursor cell marker gene. The probe, the primer, the primer set, or the antibody may be labeled. By any of the hybrid formation method, the nucleic acid amplification method, and the antigen-antibody reaction method, the detection kit further detects the expression of the dopaminergic neuron proliferative progenitor cell marker gene except for the Msx1 genes and the Msx2 genes, or the expression of the postmitotic dopaminergic neuron precursor cell marker gene.

### [Screening method]

The detection method according to the present invention can be applied to screening for effective substances for inducing differentiation into the dopaminergic neuron proliferative progenitor cells. Specifically, effective substances for inducing differentiation into dopaminergic neuron proliferative progenitor cells can be screened for by determining whether or not the addition of a substance to be tested has induced the differentiation into the dopaminergic neuron proliferative progenitor cells using expression of an Msx1 gene or an Msx2 gene as an index.

Therefore, the present invention provides a method for screening for an effective substance for inducing differentiation into a dopaminergic neuron proliferative progenitor cell, comprising the following steps of:
(i) contacting a cell that can differentiate into a dopaminergic neuron proliferative progenitor cell, with a substance to be tested; and
(ii) detecting expression of an Msx1 gene or an Msx2 gene in the cell contacted with the substance to be tested.

The cell that can differentiate into a dopaminergic neuron proliferative progenitor cell in step (i) can be preferably collected from an embryonic midbrain ventral region or from culture cells containing neuron progenitor cells induced to differentiate from ES cells.

"Contacting with a substance to be tested" in step (i) can be performed by, for example, adding the substance to be tested to culture cells containing the cells that can differentiate into a dopaminergic neuron proliferative progenitor cell.

"Substance to be tested" includes a synthesized low-molecular compound, a protein, a synthesized peptide, a purified or partially purified polypeptide, an antibody, a bacterium-releasing material (comprising bacterial metabolite), and a nucleic acid (such as antisense, ribozyme, and RNAi), and is preferably a compound or a salt thereof, or a solvate (such as hydrate) thereof, but is not limited thereto. "Substance to be tested" may be a novel substance or a known substance.

In step (ii), according to the detection method of the present invention, the expression of the Msx1 gene or the Msx2 gene can be detected.

Specifically, steps (a) and (b) are performed for the detection by utilizing the hybridization method. Steps (c) and (d) are performed for the detection by utilizing the nucleic acid amplification method. Steps (e) and (f) are performed for the detection by utilizing the antigen-antibody reaction. Thus, the expression of the Msx1 gene and the Msx2 gene can be detected.

In step (ii), when the expression of the Msx1 gene and the Msx2 gene is detected in the cell sample by contacting the substance to be tested, the substance can be determined as the effective substance for inducing differentiation into the dopaminergic neuron proliferative progenitor cells.

The substance specified by the screening method according to the present invention can be used as the effective substance for inducing differentiation into the dopaminergic neuron proliferative progenitor cells.

The present invention provides the method for screening for an effective substance for inducing differentiation into a dopaminergic neuron proliferative progenitor cell, further comprising the step of:
(iii) detecting expression of the dopaminergic neuron proliferative progenitor cell marker gene except for the Msx1 genes and the Msx2 genes.

When the expression of the Msx1 gene or the Msx2 gene is detected in step (ii) and the expression of the dopaminergic neuron proliferative progenitor cell marker gene except for the Msx1 genes and the Msx2 genes is detected in step (iii), the substance can be determined as the effective substance for inducing differentiation into dopaminergic neuron proliferative progenitor cells, with high accuracy.

Step (iii) may be performed after step (i) and may be performed before or after the step (ii).

"The dopaminergic neuron proliferative progenitor cell marker gene except for the Msx1 genes and the Msx2 genes" includes the dopaminergic neuron proliferative progenitor cell marker gene except for the Msx1 genes and the Msx2 genes that are expressed in the midbrain most ventral ventricular region (VZ region), and, for example, includes an Lrp4 gene, a Nato3 gene, and a Mash1 gene.

The detection of the dopaminergic neuron proliferative progenitor cell marker gene except for the Msx1 genes and the Msx2 genes is not particularly limited as long as using a method by which the expression of the known gene can be detected, for example, includes the above-described hybridization method, the nucleic acid amplification method, and the antigen-antibody reaction method.

### [Production Method]

The detection method according to the present invention can detect or select the dopaminergic neuron proliferative progenitor cells. The dopaminergic neuron proliferative progenitor cells for use in the treatment of the Parkinson's disease. Therefore, the dopaminergic neuron proliferative progenitor cells for use in the treatment of the Parkinson's disease can be produced from the detected or selected dopaminergic neuron proliferative progenitor cells using the expression of an Msx1 gene or an Msx2 gene as an index.

The present invention provides a method for producing a dopaminergic neuron proliferative progenitor cell, comprising the steps of:
(iv) obtaining cells that can contain a dopaminergic neuron proliferative progenitor cell;
(v) detecting or selecting the dopaminergic neuron proliferative progenitor cell by using the detection method according to the present invention; and
(vi) culturing the cell detected or selected in step (v).

The present invention provides a method for treating the Parkinson's disease comprising the step of transplanting the dopaminergic neuron proliferative progenitor cells detected or selected by the detection method according to the present invention, or the dopaminergic neuron proliferative progenitor cells produced by the production method according to the present invention, into a mammal including a human.

According to the treatment method of the present invention, the transplanted dopaminergic neuron proliferative progenitor cells produce dopamine, and thus, the Parkinson's disease can be prevented and/or treated.

When the treatment method according to the present invention is performed, the cells that can contain the dopaminergic neuron proliferative progenitor cells can be collected from a mammal comprising a human, and preferably, the individual subjected to the transplantation or an aborted fetus.

The dopaminergic neuron proliferative progenitor cells can be transplanted into a brain, preferably, a midbrain.

In the present specification, "detection" includes "discrimination".

### Examples

Hereinafter, the present invention will be specifically explained by Examples, but the following Examples do not limit the scope of the present invention.

### [Example 1] Expression Analysis of Msx1 Gene and Msx2 Gene

### (1) Analysis by RT-PCR Method

In order to confirm that an Msx1 gene and an Msx2 gene are expressed in the cells of dopaminergic neuron lineage, expressions of mRNAs of Msx1, Msx2, DAT, Lmxla, and Lrp4 in each region of a mouse embryonic midbrain were investigated by a RT-PCR method according to the following protocol. Here, DAT is a marker gene of the dopaminergic neuron (Development. 2004; 131(5):1145-55.), Lmxla is a marker gene of dopaminergic neurons and dopaminergic neuron precursor cells (WO2005/052190), and Lrp4 is a marker gene of the dopaminergic neuron proliferative progenitor cells (WO2004/065599).

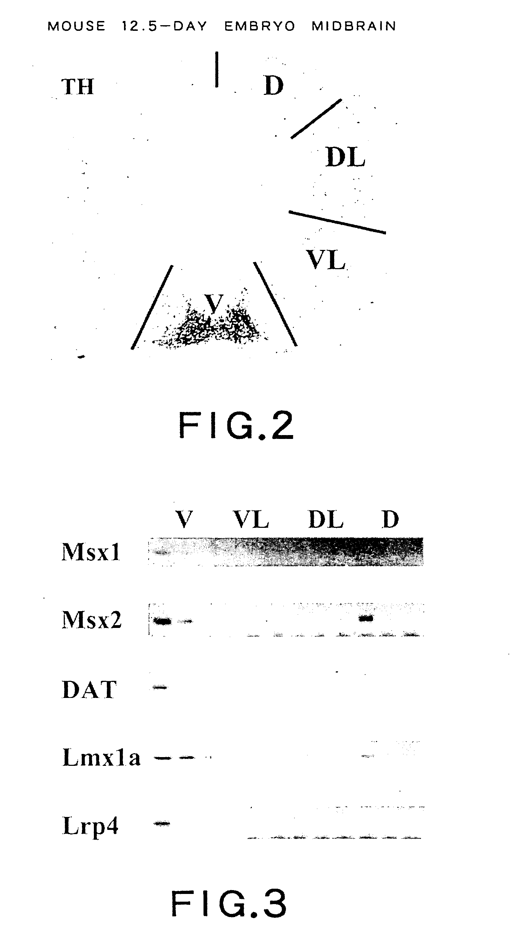

From a 12.5-day mouse (obtained from SLC) embryo, 4 regions (V region: most ventral region, VL region: ventral lateral region, DL region: dorsal lateral region, and D region: most dorsal region) of the midbrain shown in Figure 2 were cut out, and the total RNA was prepared by using a RNeasy mini kit (Qiagen), and double-strand cDNA was synthesized by using a cDNA synthesis kit (TAKARA). Next, the synthesized cDNA was digested with the restriction enzyme RsaI (TAKARA), and then, ad2 was added thereto, and PCR was performed using ad2S as a primer, and thereby, cDNA was amplified and used as a template for RT-PCR. The amplification was carried out under the conditions that incubation was performed for 5 minutes at 72°C, and then reactions for 30 seconds at 94°C, for 30 seconds at 65°C, and for 2 minutes at 72°C, were performed at 15 cycles, and finally, incubation was performed for 2 minutes at 72°C.
ad2S: cagctccacaacctacatcattccgt (SEQ ID NO:71)
ad2A: acggaatgatgt (SEQ ID NO:72)

Next, by using the cDNAs corresponding to the amplified cDNA of 4 ng, 0.4 ng, and 0.04 ng as templates, PCR was performed in the following reaction system.

| | |
|---|---|
| 10xExTaq | 1 µl |
| 2.5 mM dNTP | 0.8 µl |
| ExTaq | 0.05 µl |
| 100 µM primer | 0.1 µl for each |
| cDNA | 1 µl |
| Distilled water | 6.95 µl |

After incubation for 2 minutes at 94°C, the amplification reaction for 30 seconds at 94°C, for 30 seconds at 65°C, and for 2 minutes at 72°C, was performed, and finally, incubation was performed for 2 minutes at 72°C. The PCR amplifications were performed at 26 cycles for Lrp4, DAT, and Lmxla, at 32 cycles for Msx1, and at 27 cycles for Msx2.

The following primers were used in the PCR.
Lrp4:
   tagtctaccactgctcgactgtaacg (SEQ ID NO:73)
   cagagtgaacccagtggacatatctg (SEQ ID NO:74)
DAT:
   cagaatcctgtgctcacggtagttgc (SEQ ID NO:75)
   actaaagtggctgcaagctgaccagg (SEQ ID NO:76)
Lmxla:
   tggttcaggtgtggttccagaaccag (SEQ ID NO:77)
   tctgaggttgccaggaagcagtctcc (SEQ ID NO:78)
Msx1:
   tagcctacatgggcggtgtagagtcc (SEQ ID NO:79)
   caccgagacccaggtgaagatgatgg (SEQ ID NO:80)
Msa2:
   atatccaaccggcgtggcatagagtc (SEQ ID NO:81)
   tggttccagaaccgaagggctaaggc (SEQ ID NO:82)

As a result, it became revealed that mRNAs of the Msx1 gene and the Msx2 gene are selectively expressed in the V region and D region of the midbrain in which the marker genes of dopaminergic neuron and dopaminergic neuron precursor cells are expressed (Figure 3).

### (2) Analysis by in situ Hybridization

Furthermore, in order to investigate the expression pattern in detail, by *in situ* hybridization according to the following protocol, expression analysis of mRNA of Msx1, Nurr1, and tyrosine hydroxylase (TH) was performed. Nurr1 and TH are marker genes that are known to be induced to express first after postmitotic in the dopaminergic neuron precursor cells (Science. 1997 11; 276(5310):248-50.).

First, a DIG-probe was produced by the following method.
From a 12.5-day mouse (obtained from SLC) embryo, the midbrain afterbrain region was cut out, and the total RNA was prepared by using the RNeasy mini kit (Qiagen), and double-strand cDNA was synthesized by using the cDNA synthesis kit (TAKARA). Then, the cDNAs of Msx1, Nurr1 and TH were amplified by using the synthesized cDNAs as templates.

| | |
|---|---|
| 10xExTaq | 5 µl |
| 2.5 mM dNTP | 4 µl |
| ExTaq | 0.25 µl |
| 100 µM primer | 0.5 µl for each |
| cDNA | 1 µl |
| Distilled water | 38.75 µl |

The amplification was carried out under the conditions that incubation was performed for 5 minutes at 94°C, and then reactions for 30 seconds at 94°C, for 30 seconds at 65°C, and for 2 minutes at 72°C, were performed at 35 cycles, and finally, incubation was performed for 2 minutes at 72°C.

The following primers were used in the PCR.
Msx1:
   gccttcggcctctcttttcctcttgg (SEQ ID NO:83)
   ttcaaaagggatgcttgagagccacg (SEQ ID NO:84)
TH:
   gctgtcacgtccccaaggttcattgg (SEQ ID NO:85)
   ggagcgcatgcagtagtaagatgtgg (SEQ ID NO:86)
Nurr1:
   catatgatcgagcagaggaagacacc (SEQ ID NO:87)
   agtgcgaacaccgtagtgctgacagg (SEQ ID NO:88)

The amplified cDNA fragments were cloned into pCRII (Invitrogen) and used as templates, and thereby, DIG-probes were synthesized by the following reaction system (all of the reagents were purchased from Roche)

| | |
|---|---|
| RNA Polymerase Buffer | 2 µl |
| NTP Labeling Mix | 2 µl |
| RNase Inhibitor | 1 µl |
| RNA polymerase (T7 or SP6) | 2 µl |
| Template DNA | 1 µg |
| Distilled water | Total 20 µl |

After 2 hours at 37°C, DNaseI (Roche) treatment was performed for 15 minutes at 37°C, and the DIG-RNA probe was collected by ethanol precipitation.

Next, an 11.5-day mouse embryo was excised and fixed for 2 hours at 4°C by using 4% PFA (WAKO)/PBS (-), and then, the solution was replaced at 4°C overnight by 20% sucrose (WAKO)/PBS (-) and then the embryo was embedded with OCT (Sakura Seiki Co., Ltd.). Sections of 12 µm thickness were prepared and dried on slide glasses and then fixed again for 30 minutes at room temperature by using 4% PFA. After rinsing with PBS, hybridization (1 µg/ml DIG-RNA probe, 50% formamide (Nacalai Tesque, Inc.), 5xSSC, 1% SDS, 50 µg/ml yeast RNA (Sigma), 50 µg/ml heparin) was performed for 40 hours at 68°C. Then, rinsing (50% formamide, 5xSSC, and 1%SDS) was performed at 68°C and further rinsing (50% formamide, 5xSSC) was performed at 68°C. After rinsing with 1xTBST at room temperature, blocking (blocking agent: Roche) was performed. Alkaline phosphatase-labeled anti-DIG antibody (DAKO) was reacted at 4°C overnight, and after rinsing (1xTBST, 2mM levamisole), NBT/BCIP (DAKO) was used as the substrate for coloring.

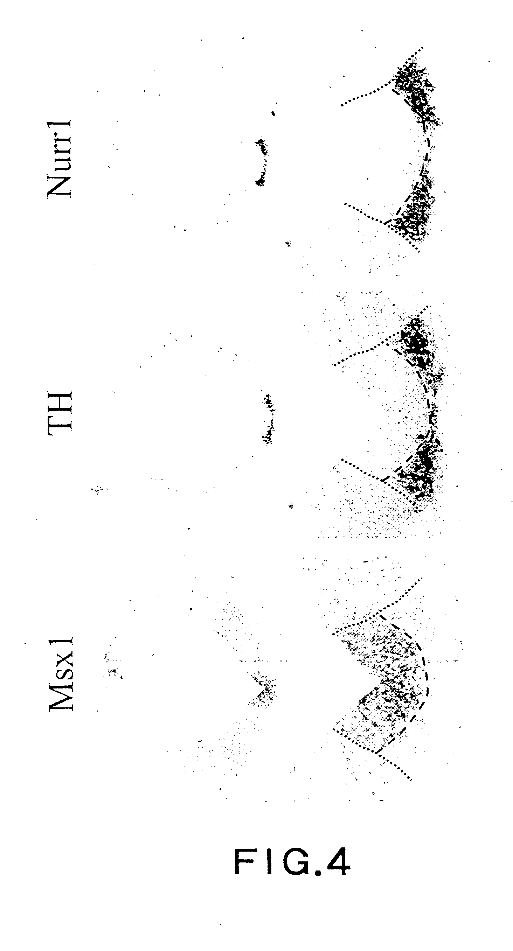

As a result, in the 11.5-day mouse embryo which is in the period of generating dopaminergic neurons, it became revealed that mRNA of Msx1 is strongly expressed in the V region in the same manner as mRNA of TH and Nurr1 (Figure 4). Also, by contrast that mRNA of TH and Nurr1 is expressed only in the mantle layer (ML) region in which the postmitotic neurons exist, it became revealed that mRNA of Msx1 is not expressed in the ML region but is expressed only in the ventricular zone (VZ) in which the proliferative progenitor cells exist (Figure 4). From the above-described results, it became revealed that mRNA of Msx1 is selectively expressed in the dopaminergic neuron proliferative progenitor cells.

### [Example 2] Expression Analysis of Msx1 Protein and Msx2 Protein

Next, by using anti-Msx1/2 antibodies (Developmental Studies Hybridoma Bank (http://www.uiowa.edu/~dshbwww/)), the expressions of Msx1/2 proteins were studied. Moreover, double staining by using an anti-Lmxla antibody was performed.

An 11.5-day mouse embryo was excised and fixed for 2 hours at 4°C by using 4% PFA (WAKO)/PBS (-), and then, the solution was replaced at 4°C overnight by 20% sucrose (WAKO)/PBS (-) and then the embryo was embedded with OCT (Sakura Seiki Co., Ltd.). Sections of 12 µm thickness were prepared, mounted on slide glasses, dried for 30 minutes at room tempreture, and then moistened again with PBS (-). Next, blocking (Blockase (Dainippon Sumitomo Pharma Co., Ltd.)) was performed for 30 minutes at room temperature, and then, reaction with a primary antibody (Developmental Studies Hybridoma Bank) was performed for one hour at room temperature, and then, reaction was further performed at 4°C overnight. By using 0.1% Tween-20/PBS (-), rinsing was performed for 15 minutes at room temperature three times. Next, a fluorescence-labeled secondary antibody (Jackson) was reacted for one hour at room temperature and rinsing was performed in the same manner, and then, rinsing with PBS(-) was performed for 10 minutes at room temperature, and sealed.

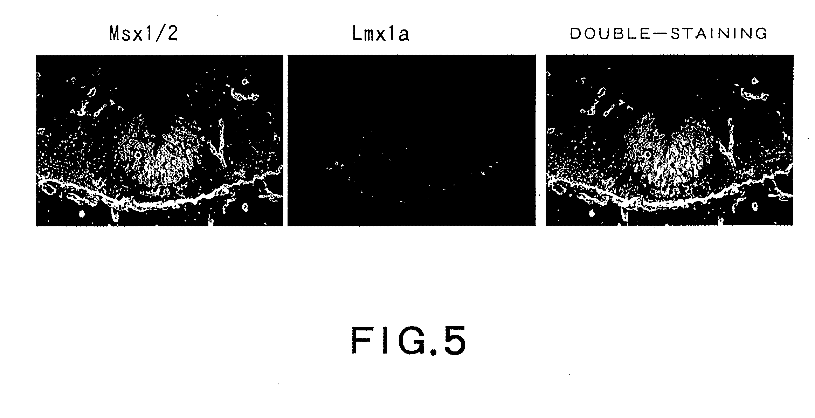

As a result, it became revealed that Msx1/2 is expressed in the midbrain of the 11.5-day mosue embryo as protein as well as mRNA (Figure 5). From the result of double staining with anti-Lmx1a antibody, it was confirmed that Msx1/2 protein is co-expressed in the same cell as Lmxla in the VZ region, and it became revealed that the expression regions also completely correspond to each other in the dorsoventral direction (Figure 5).

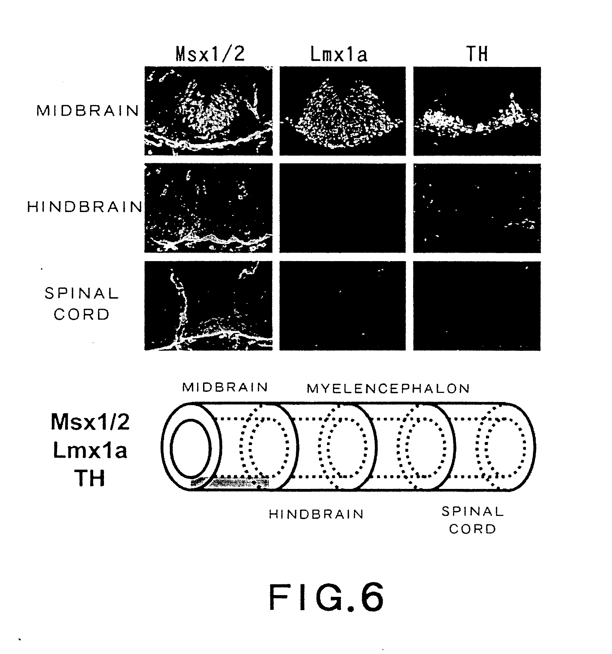

Moreover, when region specificity in the central nervous system was investigated with respect to the expressions of Msx1/2 proteins according to the above-described method, it became revealed that the Msx1/2 proteins are expressed selectively in the ventral region of the midbrain in which dopaminergic neurons are produced, particularly, in the VZ region (Figure 6).

### [Example 3] Expressions of Msx1 Gene and Msx2 Gene in Dopaminergic neurons Induced to Differentiate from ES Cells

Whether an Msx1 gene and an Msx2 gene are expressed when ES cells are induced to differentiate into dopaminergic neurons was studied.

First, according to the SDIA method (Kawasaki et al. Neuron. 2000 28(1):31-40.), ES cells (mouse CCE strain provided from Mr. Nishikawa in Riken CDB, Kawasaki et al. Neuron. 2000 28(1):31-40.) was induced to differentiate into dopaminergic neurons. The cells were collected after 4, 6, 8, 10, 12 days after the induction. The total RNA was prepared by using the RNeasy mini kit (Qiagen), and RT-PCR was performed. First, with respect to 1 µg of the total RNA, cDNA synthesis was performed by using the RNA PCR kit (TAKARA). By using the cDNAs corresponding to 10 ng, 1 ng, and 0.1 ng as templates, PCR was performed in the following reaction system.

| | |
|---|---|
| 10xExTaq | 2 µl |
| 2.5 mM dNTP | 1.6 µl |
| ExTaq | 0.1 µl |
| 100 µM primer | 0.2 µl for each |
| cDNA | 1 µl |
| Distilled water | 14.9 µl |

After incubation for 2 minutes at 94°C, the reaction for 30 seconds at 94°C, for 30 seconds at 65°C, and for 2 minutes at 72°C, was performed at 35 cycles, and finally, incubation was performed for 2 minutes at 72°C.

The following primers were used in the PCR.
TH:
   gttcccaaggaaagtgtcagagttgg (SEQ ID N0:89)
   gaagctggaaagcctccaggtgttcc (SEQ ID N0:90)
DAT:
   ctccgagcagacaccatgaccttagc (SEQ ID NO:91)
   aggagtagggcttgtctcccaacctg (SEQ ID NO:92)
Nurr1:
   cactcctgtgtctagctgccagatgc (SEQ ID NO:93)
   agtgcgaacaccgtagtgctgacagg (SEQ ID NO:94)
Ptx3:
   tgagccgcaggtctgtggatccatcc (SEQ ID NO:95)
   tccctgttcctggccttagtcctagg (SEQ ID NO:96)
En1:
   atcctccgagtggacattcacatagg (SEQ ID NO:97)
   atgtccagcaaatagagatcgctacac (SEQ ID NO:98)

In addition, for Msx1, Msx2, and Lmxla, the primers of Example 1 were used. Moreover, Ptx3 and En1 are known as markers of the postmitotic dopaminergic neuron precursor cells.

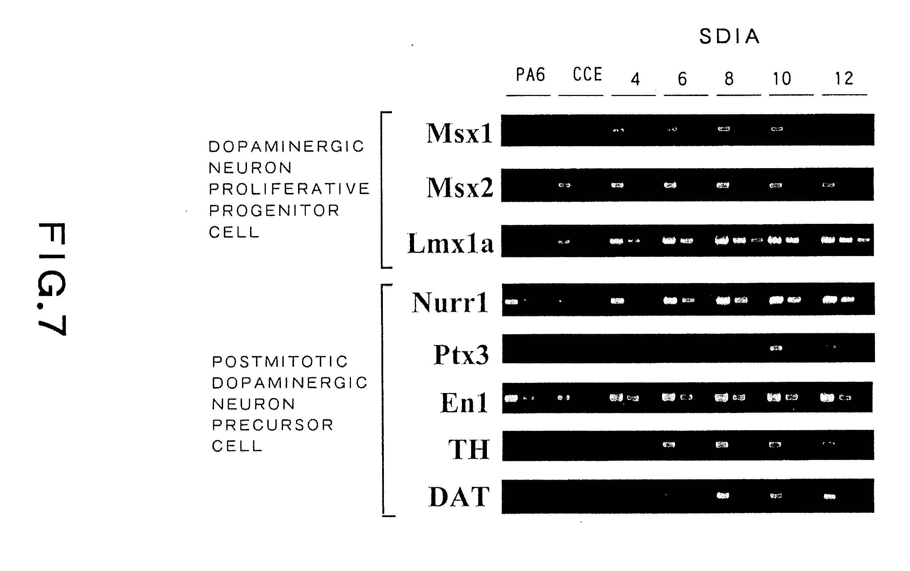

As a result, expression of mRNA of Msx1 was not recognized in ES cells (CCE), but it became revealed that as a result of the differentiation induction, the expression is induced from the fourth day in the same manner as Lmxla, which is a marker gene of the dopaminergic neuron proliferative progenitor cells (Figure 7).
On the other hand, expression of mRNA of Msx2 can also be recognized in ES cells (CCE), and it became revealed that the expression increases after the differentiation induction (Figure 7).

### [Example 4] Expression of Msx1 Gene and Msx2 Gene in Dopaminergic neuron proliferative Progenitor Cells Sorted by Lrp4

In order to confirm that an Msx1 gene and an Msx2 gene are expressed in the dopaminergic neuron proliferative progenitor cells, the dopaminergic neuron proliferative progenitor cells were separated from the cells derived from the midbrain of a 12.5-day mouse embryo and SDIA differentiation induction cells respectively using Lrp4 expressed selectively in the dopaminergic neuron proliferative progenitor cells as a marker. The expressions of mRNA of Msx1 and mRNA of Msx2 in these cells were investigated.

First, a gene sequence encoding the extracellular region (161-502 amino acids) in an Lrp4 gene was gene-transfected into 293E cells (ATCC), and the extracellular region of the Lrp4 protein was expressed and collected. A hamster (obtained from SLC) was immunized with the collected protein, and then, lymphocytic cells were extracted and cell-fused with myeloma cells (ATCC) to obtain an anti-Lrp4 antibody-producing hybridoma.

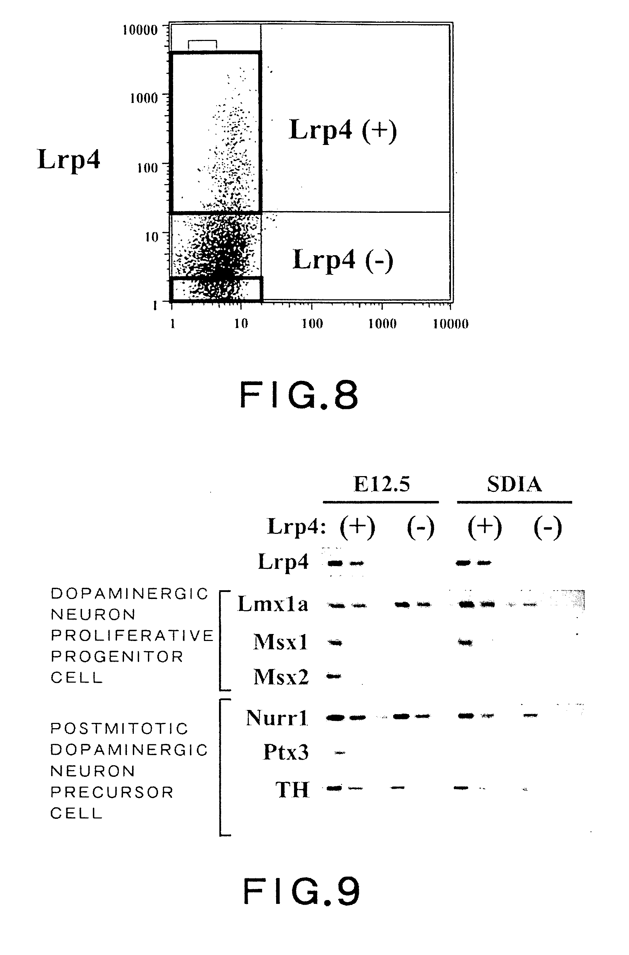

The group of the cells containing the dopaminergic neuron proliferative progenitor cells derived from the midbrain of the 12.5-day mouse embryo or induced to differentiate from ES cells *in vitro* was dispersed by using a cell dissociation buffer^{™} (Invitrogen), and then, without being subjected to fixation and permeabilization treatments, the cells were stained for 20 minutes at 4°C by using an anti-Lrp4 monoclonal antibody (the antibody produced from hybridoma (Accession No. FERM BP-10315 and FERM BP-10316)) with a culture supernatant diluted to 1/2, 1% fetal bovine serum (JRH), and 1mM EDTA/SDIA differentiation medium (Kawasaki et al. Neuron (2000)28(1):31-40). Then, with 1% fetal bovine serum and 1mM EDTA/SDIA differentiation medium, rinsing was performed for 3 minutes at 4°C three times, and the cells were stained for 20 minutes at 4°C by using a biotin-labeled anti-hamster IgG antibody (Jackson, 10 µg/ml, 1% fetal bovine serum, 1mM EDTA/SDIA differentiation medium), and then, rinsing was performed in the same manner. The cells were stained for 20 minutes at 4°C by using a PE-labeled streptavidin (Pharmingen, 20 µg/ml, 1% fetal bovine serum, 1mM EDTA/SDIA differentiation medium), and then, rinsing was performed in the same manner. After the staining, Lrp4 positive cells and negative cells were separated by a cell sorter (FACS vantage SE, Becton Dickinson) (Figure 8). The total RNA was prepared from the cells immediately after the separation, by using the RNeasy mini kit (Qiagen), and the double stand cDNA was synthesized by using the cDNA synthesis kit (TAKARA). After digestion with restriction enzyme RsaI (TAKARA), ad2 was added thereto, and ad2S was used as a primer, and the cDNA was amplified by PCR of 15 cycles and used for the template for RT-PCR. The amplification was carried out under the conditions that incubation was performed for 5 minutes at 72°C, and then reactions for 30 seconds at 94°C, for 30 seconds at 65°C, and for 2 minutes at 72°C, were performed at 15 cycles, and finally, incubation was performed for 2 minutes at 72°C.

Next, by using the cDNAs corresponding to the amplified cDNA of 4 ng, 0.4 ng, and 0.04 ng as templates, PCR was performed in the following reaction system.

| | |
|---|---|
| 10xExTaq | 1 µl |
| 2.5 mM dNTP | 0.8 µl |
| ExTaq | 0.05 µl |
| 100 µM primer | 0.1 µl for each |
| cDNA | 1 µl |
| Distilled water | 6.95 µl |

The primers having the above-described sequences were used.

After incubation for 2 minutes at 94°C, the amplification reaction for 30 seconds at 94°C, for 30 seconds at 65°C, and for 2 minutes at 72°C, was performed, and finally, incubation was performed for 2 minutes at 72°C. The amplifications of PCR were performed at 24 cycles for Lmxla and Nurr1 and at 26 cycles for the other.

As a result, in the cells derived from the midbrain of the 12.5-day mouse embryo, mRNA of Msx1 and mRNA of Msx2 were strongly expressed in a Lrp4-positive cell population (namely, dopaminergic neuron proliferative progenitor cells) (Figure 9). Also, in the SDIA differentiation induction cells, mRNA of Msx1 and mRNA of Msx2 were strongly expressed in the Lrp4-positive cell population (Figure 9). Accordingly, it was revealed that mRNA of Msx1 and mRNA of Msx2 are expressed in the dopaminergic neuron proliferative progenitor cell, in the SDIA differentiation induction cells as well as in the cells derived from the mouse embryonic midbrain. Therefore, it was revealed that the Msx1 gene and the Msx2 gene are useful markers for discriminating not only the dopaminergic neuron proliferative progenitor cells derived from the embryonic midbrain but also the dopaminergic neuron proliferative progenitor cells induced to differentiate from ES cells *in vitro.*

## Claims

1. A polynucleotide probe or polynucleotide primer for use in the detection or selection of a dopaminergic neuron proliferative progenitor cell, which can hybridize with a polynucleotide consisting of a nucleotide sequence of an Msx1 gene or an Msx2 gene, or a complementary sequence thereto.

2. The polynucleotide probe or polynucleotide primer according to claim 1, which consists of a polynucleotide comprising a sequence of at least 10 contiguous nucleotides of a nucleotide sequence of the Msx1 gene or the Msx2 gene, or a complementary sequence thereto.

3. The polynucleotide probe or polynucleotide primer according to claim 1 or 2, wherein the nucleotide sequence of the Msx1 gene or the Msx2 gene is a nucleotide sequence comprising a part or all of a nucleotide sequence selected from the group consisting of nucleotides 1-710 and 963-1713 of SEQ ID NO:1, nucleotides 1-677 and 943-1546 of SEQ ID NO:3, nucleotides 1-484 and 736-1316 of SEQ ID NO:5, nucleotides 1-612 and 864-1801 of SEQ ID NO:7, nucleotides 1-737 and 976-1724 of SEQ ID NO:9, nucleotides 1-525 and 777-1189 of SEQ ID NO:11, nucleotides 1-612 and 864-1810 of SEQ ID NO:13, nucleotides 1-754 and 994-1754 of SEQ ID NO:15, nucleotides 1-744 and 996-1935 of SEQ ID NO:16, nucleotides 1-610 and 864-1806 of SEQ ID NO:17, nucleotides 1-610 and 864-1785 of SEQ ID N0:19, nucleotides 1-1456 and 1710-1905 of SEQ ID NO:21, nucleotides 1-625 and 891-1062 of SEQ ID NO:23, nucleotides 1-709 and 963-1895 of SEQ ID NO:25, nucleotides 1-520 and 786-1310 of SEQ ID NO:27, nucleotides 1-510 and 767-1259 of SEQ ID NO:29, nucleotides 1-473 and 712-2180 of SEQ ID NO:31, nucleotides 1-468 and 707-1138 of SEQ ID NO:33, nucleotides 1-435 and 674-1143 of SEQ ID NO:35, nucleotides 1-473 and 712-1164 of SEQ ID NO:37, nucleotides 1-473 and 712-1164 of SEQ ID NO:39, nucleotides 1-473 and 712-2048 of SEQ ID NO:41, nucleotides 1-473 and 712-2182 of SEQ ID NO:43, nucleotides 1-413 and 651-804 of SEQ ID NO:45, nucleotides 1-431 and 670-2605 of SEQ ID NO:47, nucleotides 1-435 and 674-2136 of SEQ ID NO:49, nucleotides 1-778 and 1015-1452 of SEQ ID NO:51, nucleotides 1-780 and 1017-1453 of SEQ ID NO:53, nucleotides 1-370 and 609-800 of SEQ ID NO:55, nucleotides 1-29 and 267-420 of SEQ ID NO:57, nucleotides 1-1340 and 1578-1731 of SEQ ID NO:59, nucleotides 1-541 and 778-2225 of SEQ ID NO:61, nucleotides 1-404 and 651-804 of SEQ ID NO:63, nucleotides 1-632 of SEQ ID NO:65, nucleotides 1-489 and 728-1107 of SEQ ID NO:67, and nucleotides 1-487 and 708-2569 of SEQ ID NO:69.

4. The polynucleotide probe or polynucleotide primer according to any one of claims 1 to 3, which has at least 15 base lengths.

5. A polynucleotide primer set comprising two or more kinds of the polynucleotide primers according to any one of claims 1 to 4.

6. An antibody against an Msx1 protein or an Msx2 protein, or a part thereof, for use in the detection or selection of a dopaminergic neuron proliferative progenitor cell.

7. The antibody according claim 6, wherein the Msx1 protein or the Msx2 protein, or a part thereof is a protein comprising at least 6 amino acid residues or all of an amino acid sequence selected from the group consisting of amino acids 1-143 and 242-297 of SEQ ID NO:2, amino acids 1-216 and 315-370 of SEQ ID NO:4, amino acids 1-143 and 242-297 of SEQ ID NO:6, amino acids 1-149 and 248-299 of SEQ ID NO:8, amino acids 1-143 and 242-297 of SEQ ID NO:10, amino acids 1-149 and 248-303 of SEQ ID NO:12, amino acids 1-143 and 242-323 of SEQ ID NO:14, amino acids 1-143 and 242-297 of SEQ ID NO:18, amino acids 1-143 and 242-297 of SEQ ID NO:20, amino acids 1-472 and 571-634 of SEQ ID NO:22, amino acids 1-208 and 298-353 of SEQ ID NO:24, amino acids 1-143 and 242-297 of SEQ ID NO:26, amino acids 1-138 and 237-288 of SEQ ID NO:28, amino acids 1-100 and 199-242 of SEQ ID NO:30, amino acids 1-120 and 218-267 of SEQ ID NO:32, amino acids 1-120 and 218-268 of SEQ ID NO:34, amino acids 1-120 and 219-267 of SEQ ID NO:36, amino acids 1-120 and 219-267 of SEQ ID NO:38, amino acids 1-120 and 219-267 of SEQ ID NO:40, amino acids 1-120 and 219-267 of SEQ ID NO:42, amino acids 1-120 and 219-267 of SEQ ID NO:44, amino acids 1-120 and 219-267 of SEQ ID NO:46, amino acids 1-120 and 219-267 of SEQ ID NO:48, amino acids 1-119 and 218-266 of SEQ ID NO:50, amino acids. 1-121 and No. 220-268 of SEQ ID NO:52, amino acids 1-121 and 220-269 of SEQ ID NO:54, amino acids 1-9 and 99-139 of SEQ ID NO:56, amino acids 1-9 and 99-139 of SEQ ID NO:58, amino acids 1-466 and 527-576 of SEQ ID NO:60, amino acids 1-120 and 219-267 of SEQ ID NO:62, amino acids 1-120 and 219-267 of SEQ ID NO:64, amino acids 1-120 of SEQ ID NO:66, amino acids 1-112 and 211-259 of SEQ ID NO:68, and amino acids 1-112 and 211-259 of SEQ ID NO:70.

8. A method for detecting or selecting a dopaminergic neuron proliferative progenitor cell, comprising the step of detecting expression of an Msx1 gene or an Msx2 gene.

9. The method according to claim 8, wherein the step of detecting expression of the Msx1 gene or the Msx2 gene comprises the steps of:
(a) contacting a polynucleotide derived from a cell sample to be tested, with the polynucleotide probe according to any one of claims 1 to 4; and
(b) detecting a hybridization complex.

10. The method according to claim 9, wherein in step (a), mRNA prepared from the cell sample to be tested or a complementary DNA (cDNA) transcribed from the mRNA is contacted with the polynucleotide probe.

11. The method according to claim 8, wherein the step of detecting expression of the Msx1 gene or the Msx2 gene comprises the steps of:
(c) performing a nucleic acid amplification method by using a polynucleotide derived from a cell sample to be tested as a template and the polynucleotide primer according to any one of claims 1 to 4, or the polynucleotide primer set according to claim 5; and
(d) detecting a formed amplification product.

12. The method according to claim 11, wherein in step (c), mRNA prepared from the cell sample to be tested or a complementary DNA (cDNA) transcribed from the mRNA is used as a template.

13. The method according to claim 8, wherein the step of detecting expression of the Msx1 gene or the Msx2 gene comprises the steps of:
(e) contacting a protein derived from a cell sample to be tested, with the antibody according to claim 6 or 7; and
(f) detecting an antigen-antibody complex.

14. The method according to any one of claims 9 to 13, wherein the cell sample to be tested is an ES cell induced to differentiate into a dopaminergic neuron proliferative progenitor cell.

15. The method according to claim 14, wherein the differentiation induction is carried out by an SDIA method.

16. The method according to any one of claims 9 to 13, wherein the cell sample to be tested is a cell obtained from a ventral region of an embryo midbrain.

17. The method according to any one of claims 9 to 16, wherein in step (a), step (c), or step (e), a cell in which expression of the dopaminergic neuron proliferative progenitor cell marker gene except for the Msx1 genes and the Msx2 genes is detected is used as the cell sample to be tested.

18. The method according to any one of claims 9 to 16, which further comprises the step of (g) detecting expression of the dopaminergic neuron proliferative progenitor cell marker gene except for the Msx1 genes and the Msx2 genes, after step (b), step (d), or step (f).

19. The method according to claim 17 or 18, wherein the dopaminergic neuron proliferative progenitor cell marker gene except for the Msx1 genes and the Msx2 genes is at least one gene selected from the group consisting of an Lrp4 gene, a Nato3 gene, and a Mash1 gene.

20. A kit for detecting or selecting a dopaminergic neuron proliferative progenitor cell, comprising at least the polynucleotide probe according to any one of claims 1 to 4.

21. The kit according to claim 20, which further comprises a probe, a primer, a primer set, or an antibody, which can detect the expression of the dopaminergic neuron proliferative progenitor cell marker gene except for the Msx1 genes and the Msx2 genes.

22. A kit for detecting or selecting a dopaminergic neuron proliferative progenitor cell, comprising at least the polynucleotide primer according to any one of claims 1 to 4 or the polynucleotide primer set according to claim 5.

23. The kit according to claim 22, which further comprises a probe, a primer, a primer set, or an antibody, which can detect the expression of the dopaminergic neuron proliferative progenitor cell marker gene except for the Msx1 genes or the Msx2 genes.

24. A kit for detecting or selecting a dopaminergic neuron proliferative progenitor cell, comprising at least the antibody according to claim 6 or 7.

25. The kit according to claim 24, which further comprises a probe, a primer, a primer set, or an antibody, which can detect the expression of the dopaminergic neuron proliferative progenitor cell marker gene except for the Msx1 genes or the Msx2 genes.

26. A method for screening for an effective substance for inducing differentiation into a dopaminergic neuron proliferative progenitor cell, comprising the steps of:
(i) contacting a cell that can differentiate into a dopaminergic neuron proliferative progenitor cell, with a substance to be tested; and
(ii) detecting expression of an Msx1 gene or an Msx2 gene in the cell contacted with the substance to be tested.

27. The method according to claim 26, which further comprises the step of:
(iii) detecting expression of the dopaminergic neuron proliferative progenitor cell marker gene except for the Msx1 genes and the Msx2 genes, in the cell contacted with the substance to be tested.

28. The method according to claim 27, wherein the dopaminergic neuron proliferative progenitor cell marker gene except for the Msx1 genes and the Msx2 genes is at least one gene selected from the group consisting of an Lrp4 gene, a Nato3 gene, and a Mash1 gene.

29. A method for producing a dopaminergic neuron proliferative progenitor cell, comprising the steps of:
(iv) obtaining cells that can contain a dopaminergic neuron proliferative progenitor cell;
(v) detecting or selecting the dopaminergic neuron proliferative progenitor cell, by using the method according to any one of claims 8 to 19; and
(vi) culturing the cell detected or selected in step (v).

30. Use of a polynucleotide that can hybridize with a polynucleotide consisting of a nucleotide sequence of an Msx1 gene or an Msx2 gene, or a complementary sequence thereto, for the detection or selection of a dopaminergic neuron proliferative progenitor cell.

31. The use according to claim 30, wherein the polynucleotide that can hybridize consists of a sequence of at least 10 contiguous nucleotides of a nucleotide sequence of the Msx1 gene or the Msx2 gene, or a complementary sequence thereto.

32. The use according to claim 30 or 31, wherein the nucleotide sequence of the Msx1 gene or the Msx2 gene is a nucleotide sequence comprising a part or all of a nucleotide sequence selected from the group consisting of nucleotides 1-710 and 963-1713 of SEQ ID NO:1, nucleotides 1-677 and 943-1546 of SEQ ID NO:3, nucleotides 1-484 and 736-1316 of SEQ ID NO:5, nucleotides 1-612 and No. 864-1801 of SEQ ID NO:7, nucleotides 1-737 and No. 976-1724 of SEQ ID NO:9, nucleotides 1-525 and 777-1189 of SEQ ID NO:11, nucleotides 1-612 and 864-1810 of SEQ ID NO:13, nucleotides 1-754 and 994-1754 of SEQ ID N0:15, nucleotides 1-744 and 996-1935 of SEQ ID N0:16, nucleotides 1-610 and 864-1806 of SEQ ID NO:17, nucleotides 1-610 and 864-1785 of SEQ ID NO:19, nucleotides 1-1456 and 1710-1905 of SEQ ID NO:21, nucleotides 1-625 and 891-1062 of SEQ ID NO:23, nucleotides 1-709 and 963-1895 of SEQ ID NO:25, nucleotides 1-520 and 786-1310 of SEQ ID NO:27, nucleotides 1-510 and 767-1259 of SEQ ID NO:29, nucleotides 1-473 and 712-2180 of SEQ ID NO:31, nucleotides 1-468 and 707-1138 of SEQ ID NO:33, nucleotides 1-435 and 674-1143 of SEQ ID NO:35, nucleotides 1-473 and 712-1164 of SEQ ID NO:37, nucleotides 1-473 and 712-1164 of SEQ ID NO:39, nucleotides 1-473 and 712-2048 of SEQ ID NO:41, nucleotides 1-473 and 712-2182 of SEQ ID NO:43, nucleotides 1-413 and 651-804 of SEQ ID NO:45, nucleotides 1-431 and 670-2605 of SEQ ID NO:47, nucleotides 1-435 and 674-2136 of SEQ ID NO:49, nucleotides 1-778 and 1015-1452 of SEQ ID NO:51, nucleotides 1-780 and 1017-1453 of SEQ ID NO:53, nucleotides 1-370 and 609-800 of SEQ ID NO:55, nucleotides 1-29 and 267-420 of SEQ ID NO:57, nucleotides 1-1340 and 1578-1731 of SEQ ID NO:59, nucleotides 1-541 and 778-2225 of SEQ ID NO:61, nucleotides 1-404 and 651-804 of SEQ ID NO:63, nucleotides 1-632 of SEQ ID NO:65, nucleotides 1-489 and 728-1107 of SEQ ID NO:67, and nucleotides 1-487 and 708-2569 of SEQ ID NO:69.

33. The use according to any one of claims 30 to 32, wherein the polynucleotide that can hybridize has at least 15 base lengths.

34. Use of an antibody against an Msx1 protein or an Msx2 protein, or a part thereof, for the detection or selection of a dopaminergic neuron proliferative progenitor cell.

35. The use according to claim 34, wherein the Msx1 protein or the Msx2 protein, or a part thereof is a protein comprising at least 6 amino acid residues or all of an amino acid sequence selected from the group consisting of amino acids 1-143 and 242-297 of SEQ ID NO:2, amino acids 1-216 and 315-370 of SEQ ID NO:4, amino acids 1-143 and 242-297 of SEQ ID NO:6, amino acids 1-149 and 248-299 of SEQ ID NO:8, amino acids 1-143 and 242-297 of SEQ ID NO:10, amino acids 1-149 and 248-303 of SEQ ID N0:12, amino acids 1-143 and 242-323 of SEQ ID N0:14, amino acids 1-143 and 242-297 of SEQ ID NO:18, amino acids 1-143 and 242-297 of SEQ ID NO:20, amino acids 1-472 and 571-634 of SEQ ID NO:22, amino acids 1-208 and 298-353 of SEQ ID NO:24, amino acids 1-143 and 242-297 of SEQ ID NO:26, amino acids 1-138 and 237-288 of SEQ ID NO:28, amino acids 1-100 and 199-242 of SEQ ID NO:30, amino acids 1-120 and 218-267 of SEQ ID NO:32, amino acids 1-120 and 218-268 of SEQ ID NO:34, amino acids 1-120 and 219-267 of SEQ ID NO:36, amino acids 1-120 and 219-267 of SEQ ID NO:38, amino acids 1-120 and 219-267 of SEQ ID NO:40, amino acids 1-120 and 219-267 of SEQ ID NO:42, amino acids 1-120 and 219-267 of SEQ ID NO:44, amino acids 1-120 and 219-267 of SEQ ID NO:46, amino acids 1-120 and 219-267 of SEQ ID NO:48, amino acids 1-119 and 218-266 of SEQ ID NO:50, amino acids 1-121 and 220-268 of SEQ ID NO:52, amino acids 1-121 and 220-269 of SEQ ID NO:54, amino acids 1-9 and 99-139 of SEQ ID NO:56, amino acids 1-9 and 99-139 of SEQ ID NO:58, amino acids 1-466 and 527-576 of SEQ ID NO:60, amino acids 1-120 and 219-267 of SEQ ID NO:62, amino acids 1-120 and 219-267 of SEQ ID NO:64, amino acids 1-120 of SEQ ID NO:66, amino acids 1-112 and 211-259 of SEQ ID NO:68, and amino acids 1-112 and 211-259 of SEQ ID NO:70.
